# EUROPEAN PATENT SPECIFICATION

(11) **EP 4 540 258 B1**
(45) Date of publication and mention of the grant of the patent: **15.04.2026**
(21) Application number: 23734224.1
(22) Date of filing: 20.06.2023
(51) Int. Cl.: C07B 37/04, C07D 213/12, C07D 213/24, C07D 239/26, C07D 239/74, C07D 245/06, C07D 401/14, C07D 403/10, C07D 405/10, C07D 417/10, C07D 401/10, C07D 215/06, C07J 31/00, C07J 53/00, C07D 413/14, C07F 15/00, C07J 9/00, C07J 43/00, C07J 1/00

(54) **RUTHENIUM COMPLEXES AND THEIR USES IN CHEMICAL REACTIONS**
RUTHENIUMKOMPLEXE UND IHRE VERWENDUNG IN CHEMISCHEN REAKTIONEN
COMPLEXES DE RUTHÉNIUM ET LEURS UTILISATIONS DANS DES RÉACTIONS CHIMIQUES

(30) Priority: 20.06.2022 GB 202209051
(43) Date of publication of application: 23.04.2025
(73) Proprietor: The University of Manchester, Manchester M13 9PL (GB)
(72) Inventor: LARROSA, Igor, Manchester M13 9PL (GB); SIMONETTI, Marco, Manchester M13 9PL (GB); MCARTHUR, Gillian, Manchester M13 9PL (GB)
(74) Representative: Gilani, Anwar
(86) International application number: PCT/EP2023/066681
(87) International publication number: WO 2023/247563

(56) References cited:
- MART�N MARTA ET AL: "Water-Soluble Triisopropylphosphine Complexes of Ruthenium(II): Synthesis, Equilibria, and Acetonitrile Hydration", ORGANOMETALLICS, vol. 28, no. 2, 19 December 2008 (2008-12-19), pages 561 - 566, XP93081934, ISSN: 0276-7333, DOI: 10.1021/om8008553
- MARTÍN MARTA ET AL: "Water-Soluble Triisopropylphosphine Complexes of Ruthenium(II): Synthesis, Equilibria, and Acetonitrile Hydration", ORGANOMETALLICS, vol. 28, no. 2, 19 December 2008 (2008-12-19), pages 561 - 566, XP093081934, ISSN: 0276-7333, DOI: 10.1021/om8008553
- SIMONETTI MARCO ET AL: "Cyclometallated ruthenium catalyst enables late-stage directed arylation of pharmaceuticals", NATURE CHEMISTRY, NATURE PUBLISHING GROUP UK, LONDON, vol. 10, no. 7, 21 June 2018 (2018-06-21), pages 724 - 731, XP036530431, ISSN: 1755-4330, [retrieved on 20180621], DOI: 10.1038/S41557-018-0062-3

## Description

### Background

Cross-coupling reactions are widespread in organic chemistry, such as Suzuki coupling reactions in which a carbon-carbon bond is formed in a Pd catalyst mediated reaction between an aryl halide and an aryl boronic acid, and Heck reactions in which a carbon-carbon bond is formed in a Pd catalyst mediated reaction between an unsaturated compound substituted with a halide and an alkene.

Such reactions require one or both starting materials to have a functionalised group such as a halide or boronic acid group and require precious metals such as palladium.

L. Guillemard, N. Kaplaneris, L. Ackermann and M. J. Johansson, "Late-stage C-H functionalization offers new opportunities in drug discovery" Nat. Rev. Chem., 2021, 5, 522-545 discloses late-stage C-H functionalization of drugs and drug-like compounds, and describes how the implementation of this can allow increased efficiency in the drug discovery process.

Simonetti, M., Cannas, D.M., Just-Baringo, X., Citorica-Yrezabal I.J. and Larossa I. "Cyclometallated ruthenium catalyst enables late-stage directed arylation of pharmaceuticals", Nature Chem 10, 724-731 (2018) discloses a C-H arylation catalyst of formula:

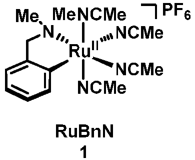

The benzylamine ligand is included in view of a proposed reaction mechanism in which a substrate having a C-H bond and a nitrogen directing group forms a cyclometalating ligand with Ru(II):

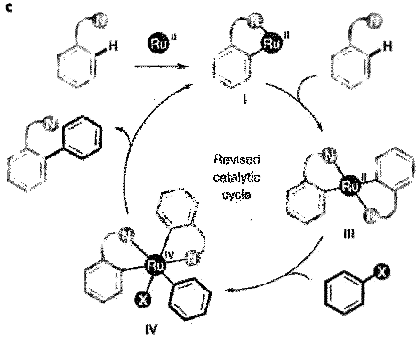

However, this Ru(II) catalyst is not air-stable.

Aebischer et al, Inorg. Chem. 1998, 37, 5915-5924, "Mechanism of Complex Formation of Ruthenium(II) Aquacomplexes with H₂C=CH₂, MeCN, Me₂SO, and CO: Metal-Water Bond Rupture as Rate-Determining Step" discloses the *in situ* formation of complexes having 1, 2 or 3 MeCN ligands formed during reaction of [Ru(H₂O)₆]²⁺ with excess acetonitrile.

It is therefore an object of the invention to provide a catalyst for conversion of a C-H bond of a substrate to a C-C bond. It is a further object of the invention to provide an air-stable catalyst for such conversions.

### Summary of the Invention

In a first aspect, the invention provides a compound of formula (I-A): 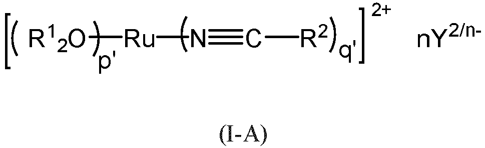
wherein R¹ in each occurrence is selected from H, optionally substituted C₁₋₁₂ alkyl and optionally substituted C₆₋₂₀ aryl;
R² in each occurrence is selected from optionally substituted C₁₋₁₂ alkyl and optionally substituted C₆₋₂₀ aryl;
p' is 1 and q' is 5 or p' is 2 and q' is 4;
p + q = 6;
Y is an anion; and
n is 1 or 2.

Optionally, p' is 1 and q' is 5.

Optionally, each R¹ is H.

Optionally, n is 2.

Optionally, Y is a borate, phosphate or perchlorate anion.

In a second aspect, the invention provides a method of forming a compound according to the first aspect, the method comprising reducing a compound of formula RuZ₃ in the presence of a compound of formula R²-CN to form an intermediate and reacting the intermediate with a compound of formula R¹₂O, wherein Z is an anionic ligand.

Optionally, Z is a halide.

Optionally, the RuZ₃ is reduced by a reducing agent. Optionally, the reducing agent is zinc.

Optionally, the RuZ₃ is reduced electrochemically.

In a third aspect, the invention provides a method of forming a compound according to any the first aspect, the method comprising reacting a compound of formula RuZp'(NC-R²)q' with a compound of formula R¹₂O wherein Z is an anionic group.

Optionally according to the third aspect, the reaction is performed the presence of a compound of formula M⁺Y⁻ wherein M⁺ is a cation

In a fourth aspect, the invention provides a method of forming a compound of formula (C), the method comprising a reaction according to Scheme 1: 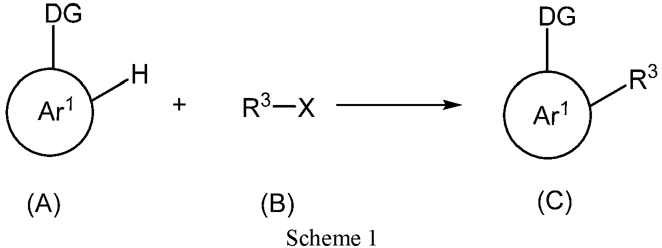 wherein (A) is a compound or a compound fragment; Ar¹ is an aromatic or heteroaromatic group which is unsubstituted or substituted with one or more substituents; DG is a directing group; R³ is an aryl, heteroaryl, alkyl, alkenyl or alkynyl group; and X is a leaving group, and wherein the reaction is catalysed by a compound of formula (I): 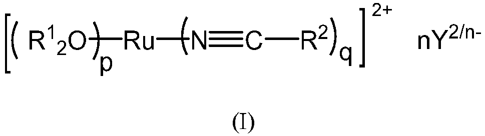
wherein R¹ in each occurrence is selected from H, optionally substituted C₁₋₁₂ alkyl and optionally substituted C₆₋₂₀ aryl;
R² in each occurrence is selected from optionally substituted C₁₋₁₂ alkyl and optionally substituted C₆₋₂₀ aryl;
p is at least 1;
q is at least 1;
p + q = 6;
Y is an anion; and
n is 1 or 2.

Optionally, the method is according to Scheme 2:

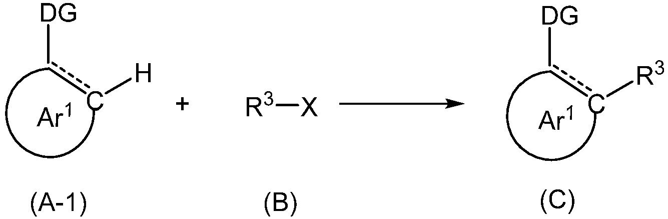

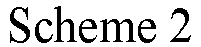

Optionally, the compound or compound fragment of formula (A) has formula (A-2) or (A-3): 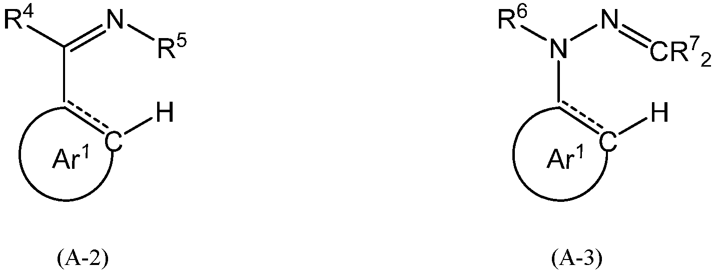 wherein R⁴ - R⁷, are each independently H or a substituent, R⁴ and R⁵ may be linked to form an optionally substituted monocyclic or fused ring; and R⁶ and R⁷ may be linked to form an optionally substituted monocyclic or fused ring.

Optionally, R⁴, R⁶ and R⁷ are each independently H, optionally substituted C₁₋₆ alkyl, optionally substituted C₆₋₂₀ aryl or -C(=O)R¹¹ wherein R¹¹ is C₁₋₆ alkyl or optionally substituted C₆₋₂₀ aryl.

Optionally, R⁵ is optionally substituted C₁₋₆ alkyl, optionally substituted C₆₋₂₀ aryl or - C(=O)R¹¹ wherein R¹¹ is C₁₋₆ alkyl or optionally substituted C₆₋₂₀ aryl.

Optionally, the compound or compound fragment of formula (A) has formula (A-4): 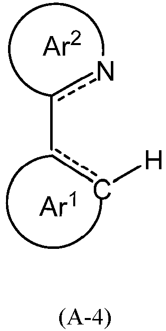 wherein Ar² is an optionally substituted monocyclic or fused heteroaromatic ring.

Optionally, Ar² has C and N ring atoms and, optionally, O or S ring atoms.

Optionally, the compound or compound fragment of formula (A) has formula (A-5) or (A-6): 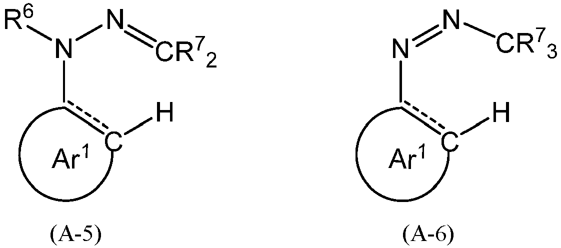 wherein R⁶ and R⁷ are each independently H or a substituent, and R⁶ and R⁷ may be linked to form an optionally substituted monocyclic or fused ring.

Optionally, the compound or compound fragment of formula (A) has formula (A-7): 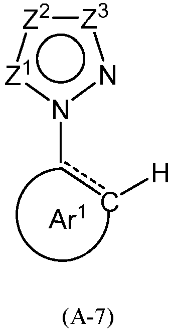 wherein Z¹, Z² and Z³ are each independently N or CR⁸ wherein R⁸ is H or a substituent.

In some embodiments, X is bound to an aromatic carbon atom of R³. In these embodiments, R³ may be an optionally substituted C₆₋₂₀ aryl, preferably optionally substituted phenyl, or optionally substituted 5-20 membered heteroaryl group.

In some embodiments, X is bound to a sp³ hybridised primary or secondary carbon atom.

In these embodiments, R³ is an optionally substituted C₁₋₁₂ alkyl.

### Drawings

The invention will be described with reference to the drawings in which:
Figure 1 is a ¹H NMR spectrum of Compound 2 according to an embodiment of the invention after exposure to air;
Figure 2A is a ¹H NMR spectrum of Comparative Compound 1 before exposure to air
Figure 2B is a ¹H NMR spectrum of Comparative Compound 1 after exposure to air; and
Figure 2C shows stacked spectra of Figures 2A and 2B.

### Detailed Description

The present inventors have found that compounds of formula (I) are air-stable and can be used for conversion of one or more C-H bonds of an aromatic carbon atom to one or more corresponding C-C bonds:

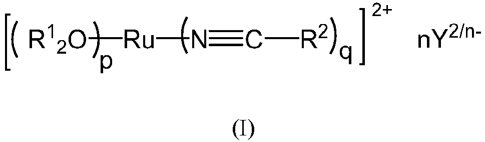

The present inventors have further found that compounds of formula (I) may be capable of catalysing such conversions at temperatures of no more than about 120°C, for example in the range of 0-120°C, optionally in the range of 0-80°C or 0-50°C.

R¹ in each occurrence may be the same or different and is selected from H, optionally substituted C₁₋₁₂ alkyl and optionally substituted C₆₋₂₀ aryl. Preferably, each R¹ is H, i.e. R¹₂O is water.

R² in each occurrence is selected from optionally substituted C₁₋₁₂ alkyl and optionally substituted C₆₋₂₀ aryl.

Each of p and q is at least 1, and p + q = 6.

Preferably, p is 1 and q is 5.

Y is an anion and n is 1 or 2. Preferably, n is 2. Y may be any suitable non-coordinating anion known to the skilled person, for example BF₄⁻,PF₆⁻ or ClO₄⁻.

A preferred compound of formula (I) is [Ru(OH₂)(R²CN)₅] 2Y⁻ wherein R² is preferably a C_{1- 6} linear, branched or cyclic alkyl group.

Without wishing to be bound by any theory, in use the R¹₂O ligand is replaced by the compound or compound fragment of formula (A) to form an intermediate which is bound to Ru through a coordinating atom of the directing group DG.

### Formula (A)

The compound or compound fragment of formula (A) consists of or comprises a fused or monocyclic aromatic or heteroaromatic ring Ar¹ bound to a directing group DG:

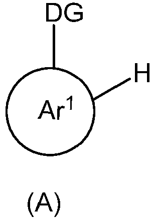

Ar¹ may be a monocyclic or fused aromatic or heteroaromatic ring, optionally a C₅₋₂₀ aromatic ring or a 5-20 membered heteroaromatic ring. A preferred Ar¹ is a benzene ring. In the case where the group of formula (A) is a compound fragment, the group of formula (A) forms part of a larger molecule, e.g. a pharmaceutical compound. It will be appreciated that the structure of the larger molecule is not particularly limited. The molecule may contain only one fragment of formula (A). The molecule may contain two or more fragments of formula (A).

The directing group DG is a group capable of coordinating to Ru of the catalyst of formula (I) and steering reaction at a carbon atom of a C-H group of Ar¹. The C atom of the C-H group is preferably *ortho* or *meta* to the carbon atom to which DG is bound. The present inventors have found that the Ru catalyst of formula (I) tends to be ortho-selective for reactions with an aromatic halide or a primary or secondary alkyl halide and meta-selective for reactions with a tertiary alkyl halide.

It will be understood that both DG and Ar¹ may be substituted with a wide range of substituents, which may be selected according to the structure of a desired end-product or intermediate. Optionally, the or each substituent has a molecular weight of less than 1000 Da.

A preferred compound or compound fragment of formula (A) has formula (A-1):

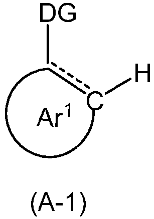

DG may comprise a coordinating group comprising a O, S or N coordinating atom, preferably a N atom.

In some embodiments, the compound or fragment of formula (A) has formula (A-2) or (A-3):

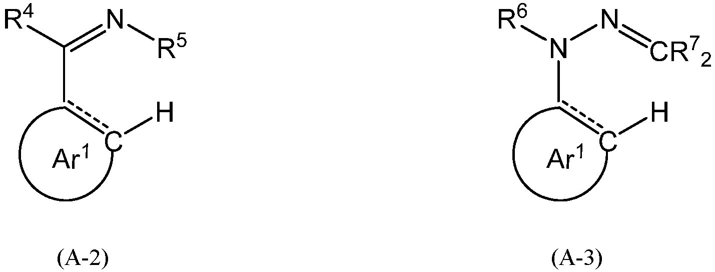

R⁴, R⁵, R⁶ and R⁷ are each independently H or a substituent.

R⁴ and R⁵ may be linked to form an optionally substituted monocyclic or fused ring.

R⁶ and R⁷ may be linked to form an optionally substituted monocyclic or fused ring.

- - - - represents a carbon-carbon single bond or carbon-carbon double bond.

Optionally, R⁴ and R⁵ are linked to form a non-aromatic, optionally fused ring, for example: 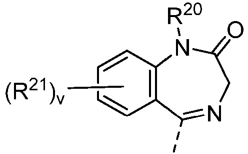 wherein R²⁰ is a C₁₋₂₀ hydrocarbyl group, preferably a C₁₋₁₂ alkyl group; R²¹ in each occurrence is a substituent, optionally a halogen, more preferably Cl; and v is 0, 1, 2, 3 or 4.

Optionally, R⁴ and R⁵ are linked to form an optionally substituted monocyclic or fused heteroaryl of formula (A-4):

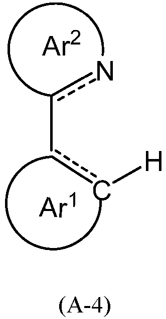

Ar² is a fused or monocyclic heteroaromatic ring.

Ar² may be a monocyclic or fused heteroaromatic ring, optionally a 5-20 membered heteroaromatic ring. Preferred heteroaromatic rings have C and N ring atoms and, optionally, O or S ring atoms.

The compound of formula (A) is optionally a compound of formula (A-5) or (A-6):

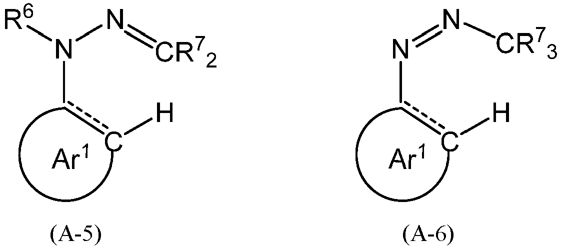

Optionally, R⁶ and R⁷ of (A-5) are linked to form an optionally substituted monocyclic or fused heteroaryl of formula (A-7) 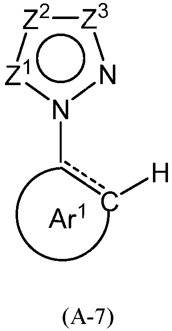 wherein Z¹, Z² and Z³ are each independently N or CR⁸ wherein R⁸ is H or a substituent.

Exemplary DG groups include, without limitation: 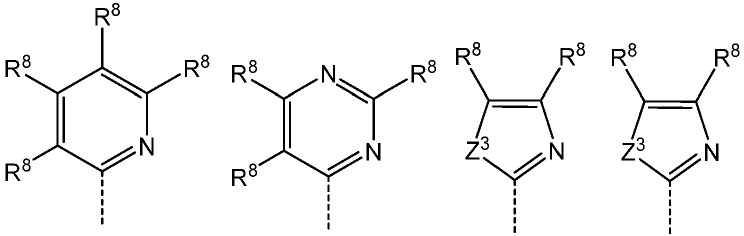 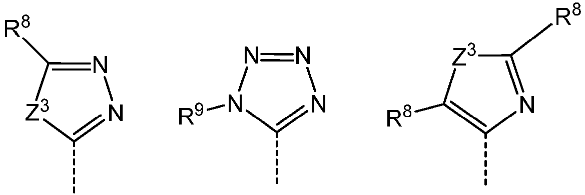 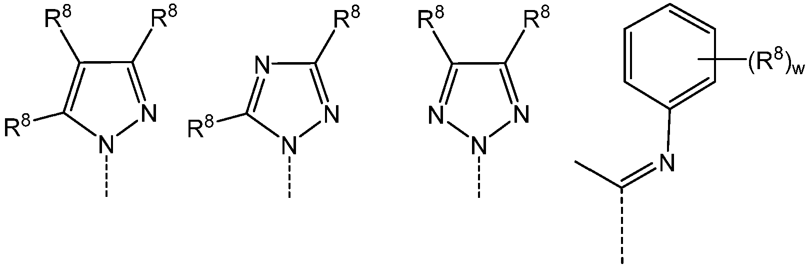 wherein Z³ is O, S or NR⁹; R⁸ in each occurrence is independently H or a substituent; R⁹ is H or a substituent; w is at least 1, optionally 1, 2, 3, 4 or 5; and two R⁸ groups bound to the same C atom or bound to adjacent C atoms or an R⁸ group and an R⁹ group bound to adjacent C and N atoms may be linked to form an unsubstituted or substituted ring.

Optionally, R⁸ in each occurrence is independently selected from H, F, optionally substituted phenyl, optionally substituted C₁₋₁₂ alkyl, OR³⁰, SR³⁰, COOR³⁰, C(=O)R³⁰, CONR³⁰ or NR³⁰ wherein R³⁰ in each occurrence is H; or an optionally substituted monocyclic or fused C₆₋₁₂ aryl, preferably optionally substituted phenyl, or an optionally substituted monocyclic or fused C₅₋₂₀ heteroaryl group.

Optionally, R⁹ is H; branched, linear or cyclic C₁₋₁₂ alkyl; or optionally substituted C₆₋₁₂ aryl or optionally substituted C₅₋₂₀ heteroaryl.

Optional substituents of an optionally substituted alkyl group as described anywhere herein include F, CN, NO₂, or an optionally substituted aryl or heteroaryl group.

Optional substituents of an optionally substituted aryl or heteroaryl group as described anywhere herein include F, Cl, CN, NO₂, or C₁₋₁₂ alkyl in which one or more non-adjacent C atoms of the C₁₋₁₂ alkyl may be replaced with O, S, COO, C(=O), CONR³¹ or NR³¹ wherein R³¹ in each occurrence is H or a C₁₋₂₀ hydrocarbyl group.

Two R⁸ groups linked to adjacent carbon atoms or an R⁸ group and an R⁹ group bound to adjacent C and N atoms may be linked to form an optionally substituted benzene, or azine ring, such as a pyridine or diazine ring, for example:

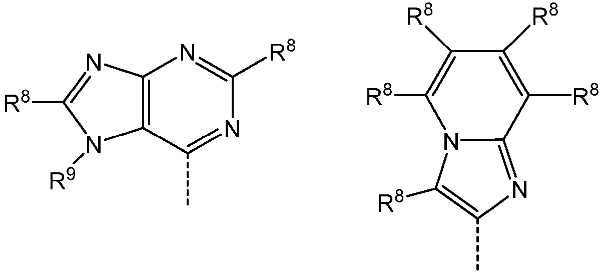

For simplicity, the compound or compound fragment of formula (A) as illustrated above shows only one C-H group, however it will be appreciated that two C-H groups may be available for reaction, for example two ortho C-H groups as illustrated below:

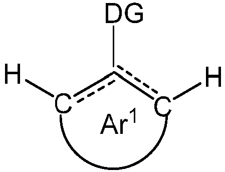

### Compound B

Compound B has formula R³-X.

X is bound to a carbon atom of R³, preferably an sp³-hybridised, primary or secondary carbon atom or an sp²-hybridised carbon atom of an aromatic or heteroaromatic ring.

It will be understood that a wide range of R³ groups may be used, according to the desired product of the reaction between compounds A and B, and as such other constituent atoms or units of R³ are not particularly limited.

It will be appreciated that a wide range of optional substituents may be selected according to the desired product. R³ may be selected from, without limitation, optionally substituted C₁₋₄₀ alkyl; optionally substituted C₂₋₄₀ alkenyl; optionally substituted C₂₋₄₀ alkynyl; optionally substituted C₆₋₂₀ aryl, preferably optionally substituted phenyl; and an optionally substituted 5-20 membered heteroaromatic group, wherein one or more non-adjacent -CH₂- groups of the C₁₋₁₂ alkyl, C₂₋₁₂ alkenyl, or C₂₋₁₂ alkynyl may be replaced with O, S, COO, CO, CONR³¹ or NR³. . Where present, the or each substituent preferably has a molecular weight of less than 1000 Da.

In the case where R³ is an optionally substituted C₁₋₁₂ alkyl group, R³-X may be a primary, secondary (including cyclic) or tertiary optionally substituted alkyl group, preferably a primary or secondary optionally substituted alkyl group.

X is a leaving group, preferably Cl, Br or I, preferably Br; a pseudohalid, preferably a sulfonate, for example tosylate or triflate; or a group of formula -N(R¹⁰)₃⁺ An⁻ wherein R¹⁰ in each occurrence is a substituent and An⁻ is an anion.

Preferably, each R¹⁰ is independently selected from the group consisting of optionally substituted C₁₋₁₂ alkyl, optionally substituted aryl e.g. optionally substituted phenyl, more preferably selected from C₁₋₁₂ alkyl and phenyl which is optionally substituted with one or more substituents selected from F, C₁₋₆ alkyl and C₁₋₆ fluoroalkyl.

An⁻ may be any suitable anion, for example a halide or a sulfonate such as triflate.

Many known compounds, including but not limited to pharmaceuticals or intermediates thereof, contain an aromatic C-H group adjacent to a group capable of acting as a directing group.

Examples of Compound B include, without limitation:

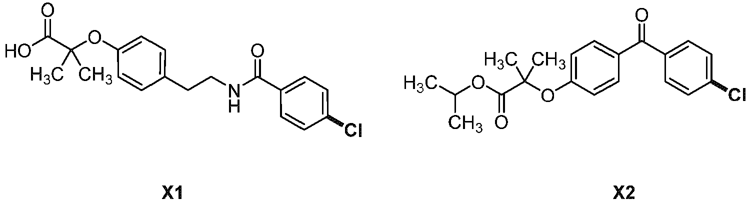

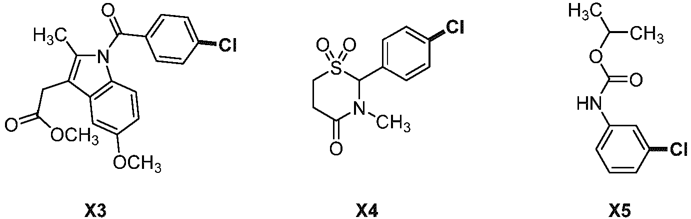

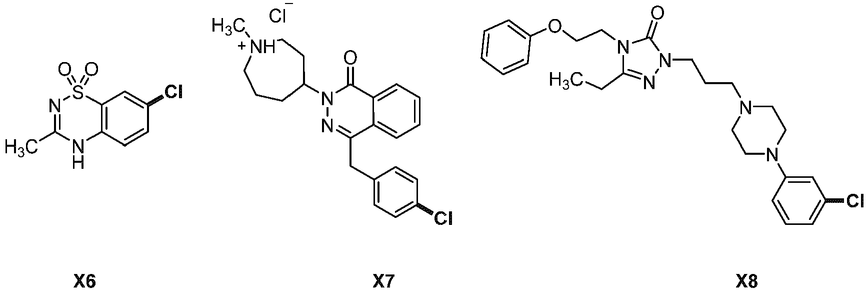

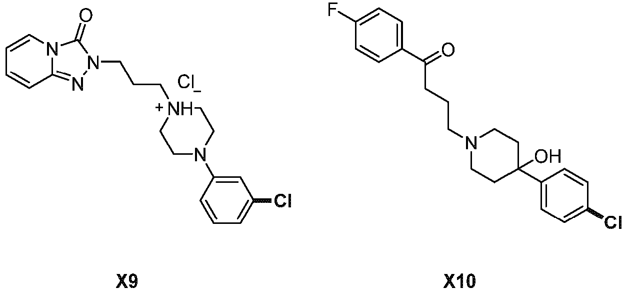

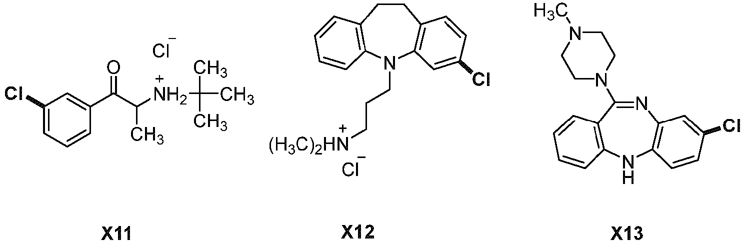

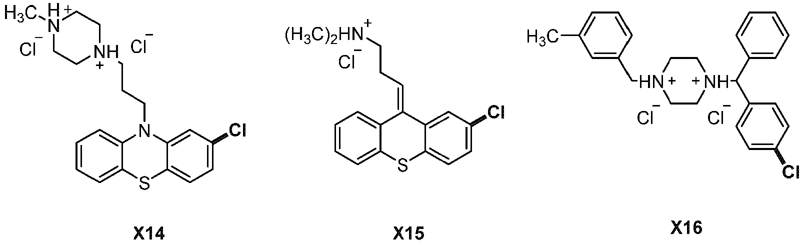

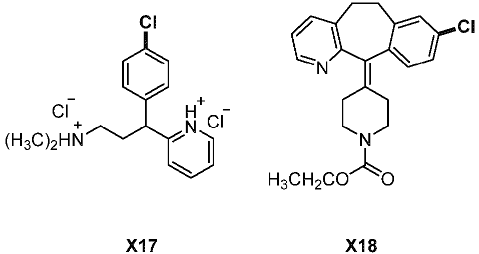

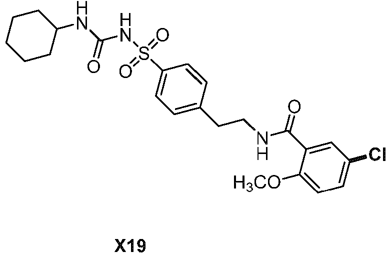

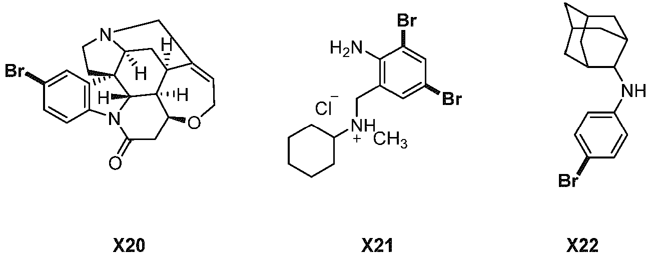

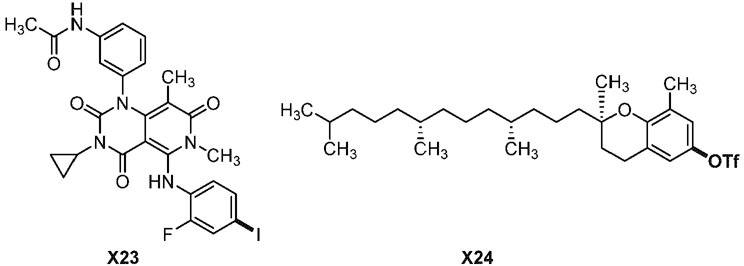

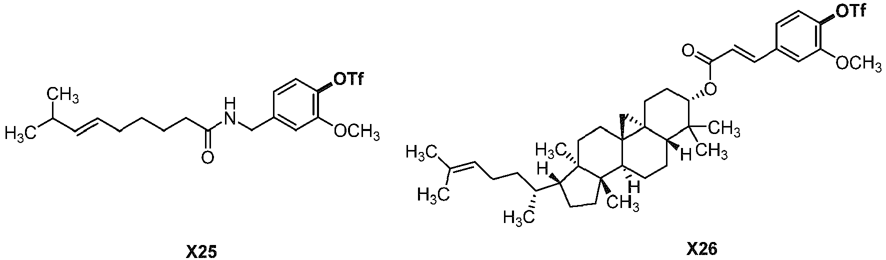

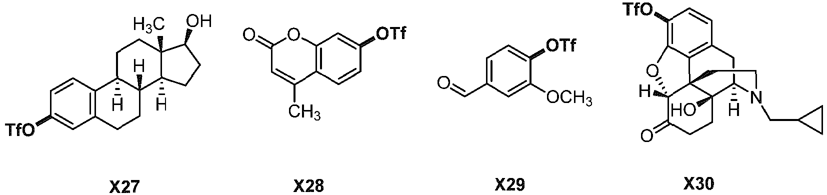

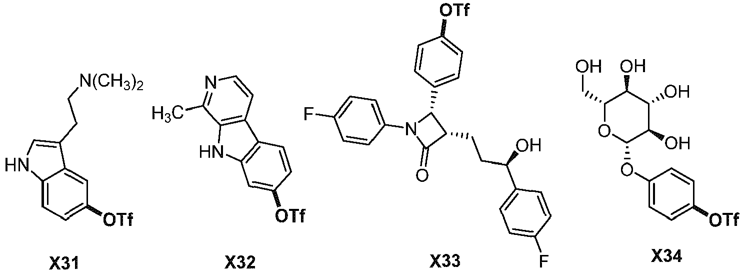

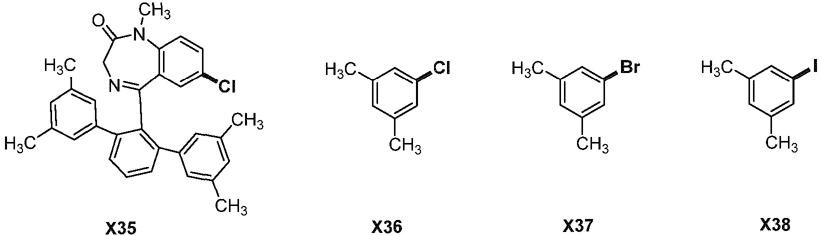

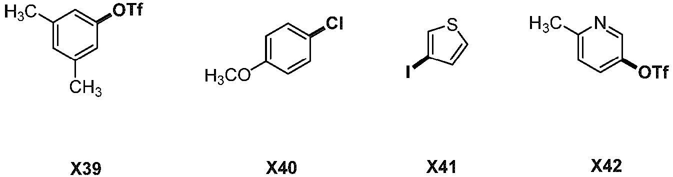

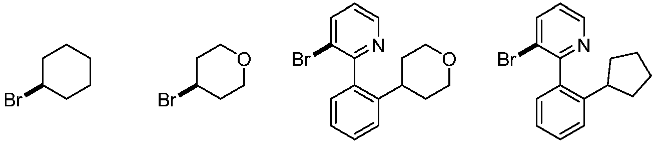

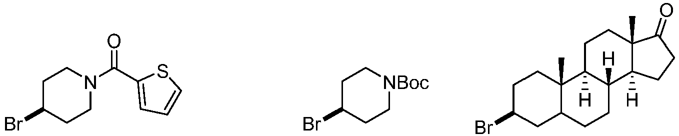

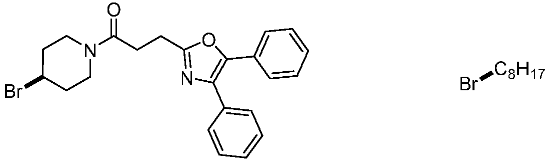

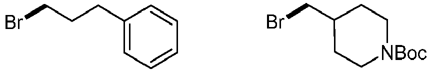

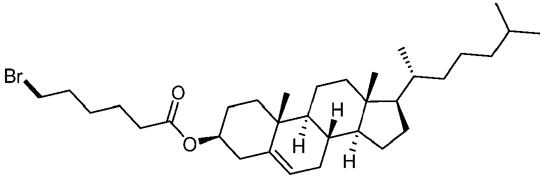

Examples of pharmaceuticals which may be formed by a method as described herein include the following, in which bonds shown with a dashed line extending through them are formed upon functionalisation of a C-H bond as described herein:

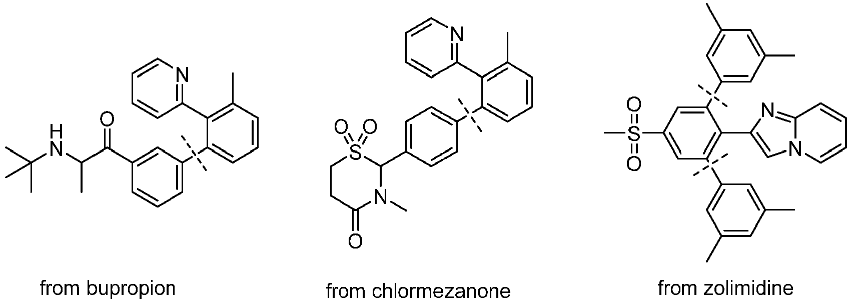

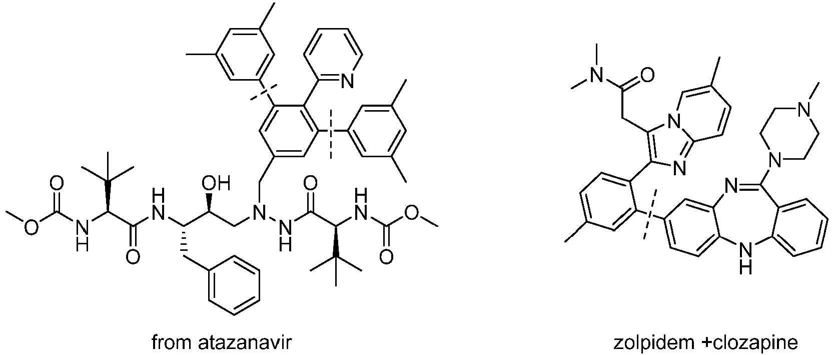

Examples of products of a reaction between a compound having a primary alkyl halide group and a known compound include the following:

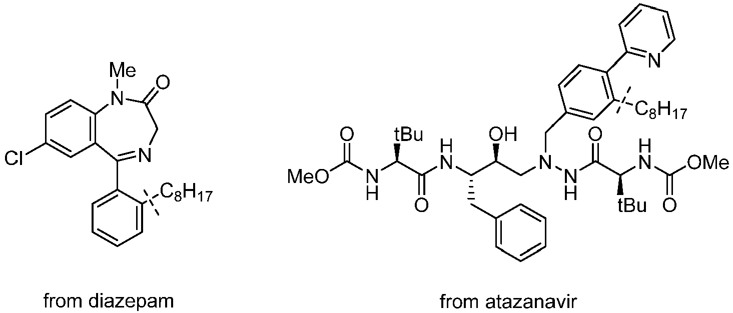

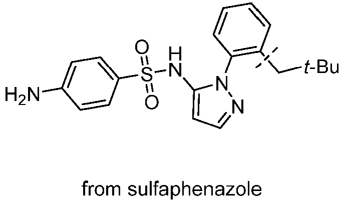

Examples of products of a reaction between a compound having a secondary alkyl halide group and a known compound include the following:

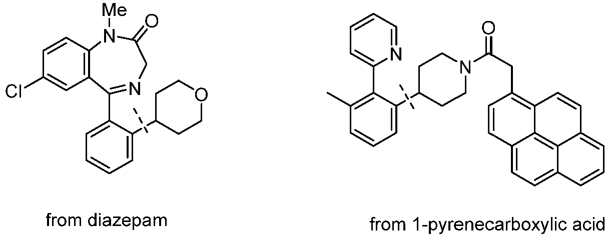

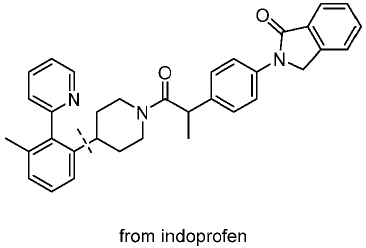

An optionally substituted C₆₋₂₀ aryl group as described herein with reference to a compound of formula (I), a compound of formula (I-A), Compound A or Compound B is preferably a C₆₋₁₂ aryl, e.g. phenyl. The C₆₋₂₀ aryl group may be, without limitation, a single monocyclic ring; a fused monocyclic aromatic group; or combinations thereof linked by a single bond.

### Catalyst synthesis

One method of forming a catalyst as described herein is reduction of a Ru³⁺ compound in the presence of R²-CN to form an intermediate which is then reacted with a compound of formula R¹₂O.

Reduction of a Ru³⁺ compound may be performed electrochemically or by a reducing agent capable of reducing Ru³⁺ to Ru²⁺. An exemplary reducing agent is an elemental metal capable of reducing Ru³⁺ to Ru²⁺, for example zinc.

The Ru³⁺ compound may be, for example, a Ru (III) halide, e.g. RuBr₃, RuCl₃ or RuI₃.

Another method of forming a catalyst as described herein is replacement of an anionic ligand Z in a compound RuZp'(NC-R²)q' with a compound of formula R¹₂O. The reaction may be performed in the presence of a compound of formula M⁺Y⁻ for providing a counterion Y⁻ of the catalyst, wherein M⁺ is a cation, e.g. a metal ion. In the case where p' is 1 and q' is 5, it will be appreciated that RuZp'(NC-R²)q' is cationic and will comprise a suitable anionic counterion.

### Examples

### General Information

All the starting materials and solvents were purchased from Acros (Fisher), Aldrich (Merck), Alfa Aesar (Fisher), Fluorochem and Generon and used without further purification unless otherwise stated. Column chromatography was carried out on silica gel (particle size 40-63 µm) using flash techniques. High resolution mass spectra were performed by the School of Chemistry Mass Spectrometry Service (University of Manchester) employing a Thermo Finnigan MAT95XP spectrometer. IR spectra were recorded using a Thermo Scientific Nicolet iS5 FTIR machine, relevant bands are quoted in cm⁻¹. ¹H NMR, ¹⁹F NMR and ^{13C} NMR spectra were recorded at 400 or 500 on Bruker instruments. ¹H NMR are referenced to the residual solvent peak at 7.26 ppm (CDCl₃), 5.35 (CD₂Cl₂) or 2.05 ppm ((CD₃)₂CO). ppm values are quoted to 2 decimal places, with coupling constants (J) to the nearest 0.1 Hz. ¹³C NMR spectra were recorded at 151, 126 or 100 MHz and quoted in ppm to 1 decimal place with coupling constants (J) to the nearest 0.1 Hz. The spectra were referenced to the residual solvent peak at 77.16 ppm (CDCl₃), 5.30 ppm (CD₂Cl₂) or 39.52 ppm ((CD₃)₂CO). ¹⁹F NMR spectra recorded at 471 or 376 MHz in CDCl₃ or CD₂Cl₂ and quoted in ppm to 2 decimal places with coupling constants (J) to the nearest 0.1 Hz.

### Preparation of Ruthenium Aqua Complex 2 General Procedure A1

i) Zn dust, tBuCN, 115 °C, 2 h

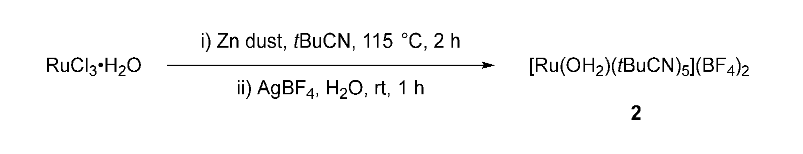

### General procedure for synthesis of ruthenium aqua catalyst 2

Pivalonitrile was dried over 4 Å molecular sieves and degassed with three freeze-pump-thaw cycles prior to use. In a glove box, RuCl₃ •×H₂O (Reagent Grade purity ≥ 95%, 1 equiv based on anhydrous molecular weight), glove-boxed stored Zn dust (<10 µm, 3.2 equiv) and pivalonitrile were loaded in an Ace pressure tube which was subsequently wrapped in Teflon tape and parafilm. The sealed tube was then taken out from the glovebox and stirred for 2 h at 115 °C ensuring the solid was being thoroughly stirred. The reaction mixture was cooled to room temperature and the pivalonitrile removed under reduced pressure. The resulting mixture was diluted with HPLC-grade water before being filtered through a small plug of Celite^{®} to remove all solids. AgBF₄ (2 equiv) was added and the reaction was vigorously stirred for 1 hour at room temperature. After this time the solution was filtered through a plug of Celite^{®} and evaporated to dryness. The residue was dissolved in acetone, filtered through a plug of Celite^{®} and evaporated to dryness. Then, it was dissolved in CH₂Cl₂, filtered through a plug of Celite^{®} and evaporated to dryness. The residue was dissolved in CH₂Cl₂ and precipitated with Et₂O affording a light-yellow solid. The solid was collected, dissolved in CH₂Cl₂ and precipitated with Et₂O. The last precipitation step was reiterated until the desired complex **2** was obtained as a fluffy light-yellow powder.
i) The General Procedure A1 was applied using 1.5 g (7.2 mmol) of RuCl₃ •×H₂O, 1.5 g (22.9 mmol) of zinc dust and 35 mL of pivalonitrile. After allowing the reaction mixture to stir and removing the pivalonitrile, HPLC grade water (150 mL) and AgBF₄ (2.8 g, 14.5 mmol) were added to and the resulting reaction mixture was allowed to stir at room temperature for 1 hour. The product was precipitated with Et₂O from a solution in DCM 5 times giving the desired complex **5** as a fluffy light-yellow solid (2.2 g, 42% yield).
ii) The General Procedure A1 was applied using 20.0 g (96.0 mmol) of RuCl₃ •×H₂O, 20.0 g (307.2 mmol) of zinc dust and 460 mL of pivalonitrile. After allowing the reaction mixture to stir and removing the pivalonitrile, HPLC grade water (2 L) and AgBF₄ (37.4 g, 192.0 mmol) were added to and the resulting reaction mixture was allowed to stir at room temperature for 1 hour. The product was precipitated with Et₂O from a solution in DCM 6 times giving the desired complex **5** as a fluffy light-yellow solid (26.3 g, 38% yield).

¹H NMR (500 MHz, CD₂Cl₂): δ 1.43 (s, 36H), 1.38 (s, 9H).

¹³C NMR (126 MHz, CD₂Cl₂): δ 135.8, 133.8, 30.8, 30.4, 28.3, 27.9.

¹⁹F NMR (376 MHz, CD₂Cl₂) : δ -150.02, -149.97.

IR (neat, cm⁻¹): 3394 (OH₂), 2279 (CN).

MS (ESI+ ): 622.2844.

HRMS (ESI+ ): Calculated for [Ru(OH₂)(*t*BuCN)₅]⁺(BF₄): 622.2848, found: 622.2848.

mp: decomposes at 180-182 °C [recrystallised by slow diffusion of diethyl ether into solution in CH₂Cl₂].

### Procedure A2: Preparation of Ruthenium Aqua Complex 2

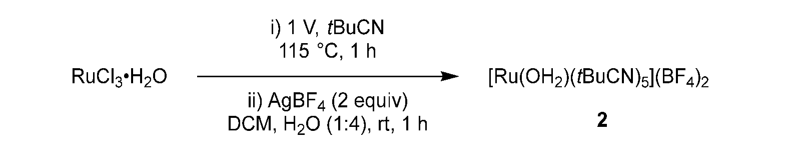

In a glovebox, an electrosyn^{®} vial was charged with RuCl₃•H₂O (100 mg, 0.48 mmol, 1 equiv), TBAPF₆ (100 mg, 0.24 mmol, 0.5 equiv) and tBuCN (4 mL). The reaction vessel was sealed, removed from the glovebox, and stirred at 115 °C on an electrosyn^{®} at constant voltage (1 V) for 1 hour. The reaction mixture was cooled to room temperature, filtered through a small plug of Celite^{®} before washing with HPLC grade water (40 mL). AgBF₄ was added (0.96 mmol, 2 equiv,) and the reaction was vigorously stirred for 1 hour at room temperature under air in darkness before filtering through a small plug of Celite^{®} and evaporating to dryness. The residue was dissolved in acetone, filtered through a small plug of Celite^{®} and evaporated to dryness. Then, it was dissolved in dichloromethane, filtered through a small plug of Celite^{®} and evaporated to dryness. The residue was dissolved in dichloromethane and precipitated with diethyl ether affording a light-yellow solid. The solid was collected, dissolved in dichloromethane, and precipitated with diethyl ether. The last precipitation step was reiterated another 3 times to give the desired aqua ruthenium complex 5 as an off white solid (20 mg, 6% yield).

### Procedure A3: Preparation of Ruthenium Aqua Complex 2

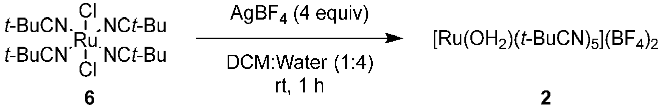

A reaction vessel fitted with a magnetic stirrer bar was charged with ruthenium chloride intermediate 6 (341 mg, 0.68 mmol, 1 equiv) and AgBF₄ (529.3 mg, 2.72 mmol, 4 equiv). 1.8 mL of DCM and 7 mL of HPLC-grade water were added and the system was allowed to stir at room temperature for 1 hour under air in darkness before filtering through a small plug of Celite^{®} and evaporating to dryness. The residue was dissolved in acetone, filtered through a small plug of Celite^{®} and evaporated to dryness. Then, it was dissolved in dichloromethane, filtered through a small plug of Celite^{®} and evaporated to dryness. The residue was dissolved in dichloromethane and precipitated with diethyl ether affording a light-yellow solid. The solid was collected, dissolved in dichloromethane, and precipitated with diethyl ether. The last precipitation step was reiterated another 3 times to give the desired aqua ruthenium complex **2** as a fine solid (85 mg, 18% yield).

Replacement of a H atom bound to an aromatic C atom was performed for a wide range compounds as set out in the following examples.

### General Procedure B: Ru-catalysed arylation of DG-containing arenes with aryl (pseudo)halides

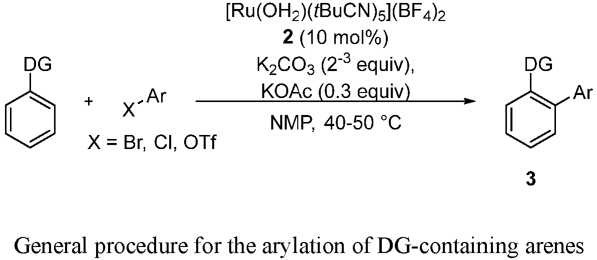

All liquid reagents were degassed with at least 3 freeze-pump-thaw cycles prior to use. KOAc and K₂CO₃ were dried at 80 °C in a vacuum oven for 48 hours prior to use. Unless otherwise indicated, a 10 mL Schlenk tube equipped with a magnetic stirring bar was charged with ruthenium aqua catalyst **2** (28.4 mg, 0.04 mmol, 10 mol%), KOAc (11.8 mg, 0.12 mmol, 30 mol%), K₂CO₃ (2-4 equiv), the appropriate DG-containing arene (0.40 mmol, 1 equiv) and the appropriate aryl halide (0.4 mmol, 1 equiv). After addition of the solids, 3 x 5 minute evac-refill cycles were performed before adding any liquid/oil reagents via injection along with the NMP (0.8 mL, 0.5 M with respect to the DG-containing arene). The vial was capped and stirred at the stated temperature for the indicated time. Upon completion, the crude mixture was loaded onto a silica gel column and purified by flash chromatography.

Data for all compounds following this procedure matches that previously reported by Larrosa *et. al.*

M. Simonetti, D. M. Cannas, X. Just-Baringo, I. J. Vitorica-Yrezabal and I. Larrosa, Nature Chem., 2018, 10, 724-731.

Reactions were performed as set out below following General Procedure B: 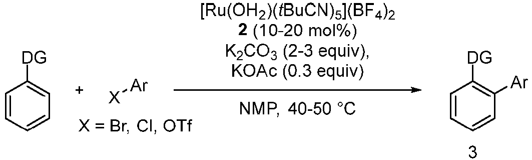 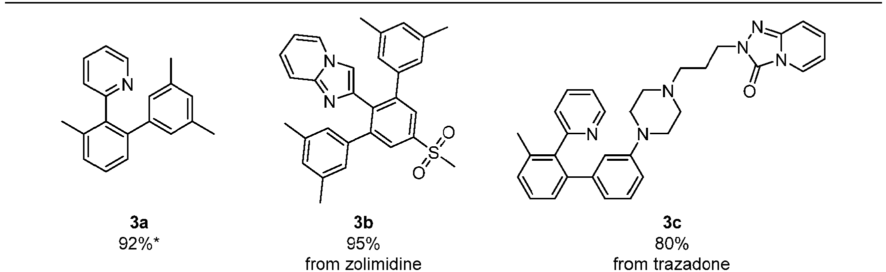 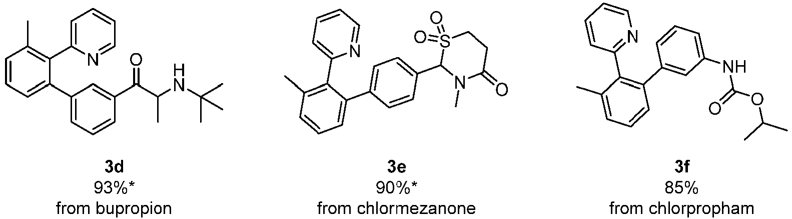 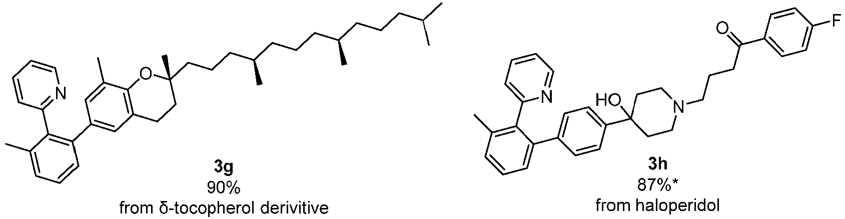 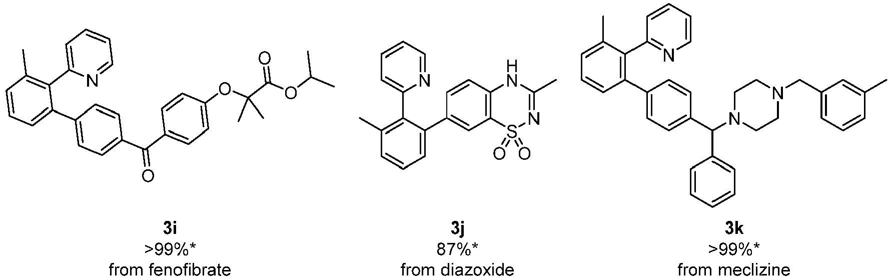 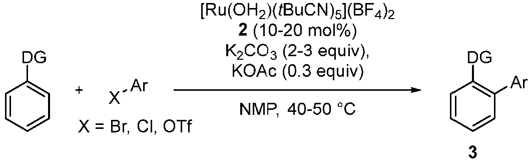 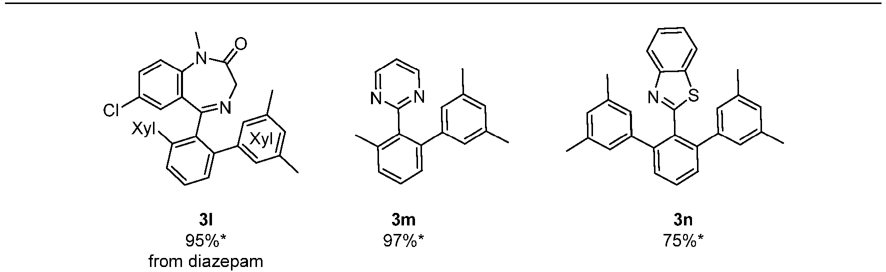 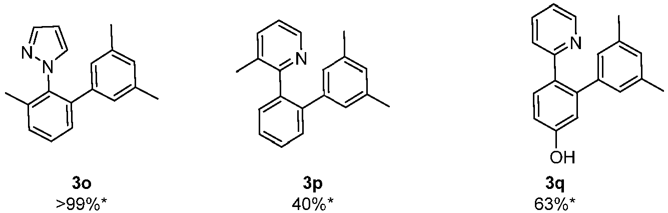 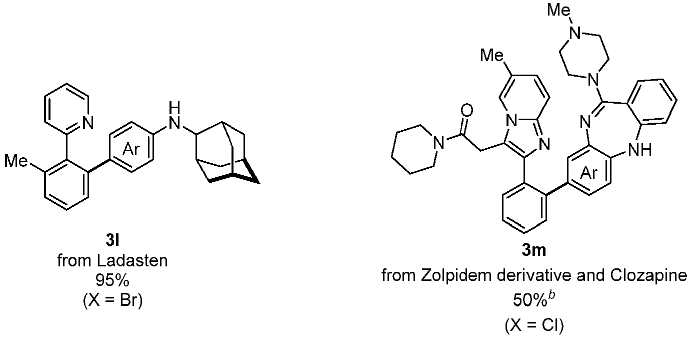 ** NMR yield calculated by ¹H Q NMR using trimethoxy benzene as internal standard.*

### Synthesis of 2-(3,3',5'-trimethyl-[1,1'-biphenyl]-2-yl)pyridine (3a)

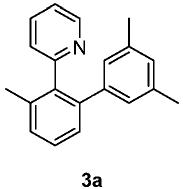

The General Procedure B was applied with 2-(o-tolyl)pyridine (67.8 mg, 0.40 mmol, 1 equiv), br-m-xylene (74 mg, 0.40 mmol, 1 equiv), K₂CO₃ (110.6 mg, 0.8 mmol, 2 equiv) and [Ru(OH₂)(*t*BuCN)₅](BF₄)₂] **2** for 3 h at 40 °C. Column chromatography eluting with 5-15% EtOAc in hexane afforded the title product **3a** as a colourless oil (100.5 mg, 92% yield).

¹H NMR (400 MHz, CDCl₃) δ 8.65 (m, 1H), 7.46 (td, *J* = 7.7, 1.8 Hz, 1H), 7.35 (m, 1H), 7.30 (m, 2H), 7.12 - 7.06 (ppm, 1H), 6.92 (d, *J* = 7.7 Hz, 1H), 6.77 (s, 1H), 6.72 (s, 2H), 2.21 (s, 3H), 2.16 (s, 6H).

¹³C NMR (101 MHz, CDCl₃) δ 159.9, 148.7, 141.5, 141.5, 139.4, 137.0, 136.6, 135.7, 129.3, 128.0, 127.9, 127.9, 127.6, 125.7, 121.2, 21.2, 20.6.

### Synthesis of [zolimidine]-[5-m-xylene] (3b)

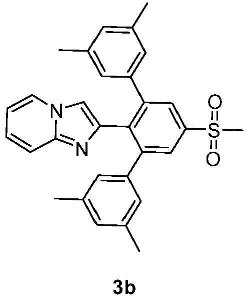

The General Procedure B was applied with Zolimidine (109.0 mg, 0.40 mmol, 1 equiv), 5-br-*m*-xylene (109.0 µL, 0.4 mmol, 2 equiv) and K₂CO₃ (165.8 mg, mmol, 3 equiv) for 72 h at 40 °C. Column chromatography eluting with 80% Et₂O in hexane afforded the title product **3b** as a pale yellow solid (176.8 mg, 95%).

¹H NMR (500 MHz, CDCl₃) δ 7.95 (s, 2H), 7.83 (dt, *J* = 6.9, 1.2 Hz, 1H), 7.44 (dd, *J* = 9.2, 1.1 Hz, 1H), 7.07 - 7.02 (m, 1H), 6.98 (s, 1H), 6.88 (s, 4H), 6.79 (s, 2H), 6.67 - 6.62 (m, 1H), 3.12 (s, 3H), 2.14 (s, 12H).

¹³C NMR (126 MHz, CDCl₃): δ 145.2, 144.0, 142.7, 140.0, 139.8, 137.5, 137.1, 128.8, 127.3, 127.2, 125.4, 124.0, 117.4, 112.3, 112.0, 44.6, 21.1.

### Synthesis of [2-(o-tolyl)pyridine]-[trazadone] (3c)

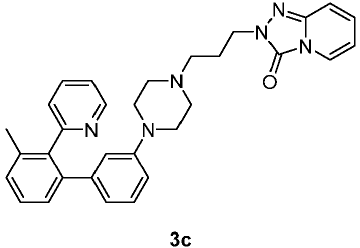

The General Procedure B was applied with 2-(o-tolyl)pyridine (67.8 mg, 0.40 mmol, 1 equiv), Trazodone • HCl (163.4 mg, 0.40 mmol, 1 equiv) and K₂CO₃ (165.8 mg, 1.2 mmol, 3 equiv) at 50 °C. Column chromatography eluting with 1-5% MeOH in CH₂Cl₂ afforded the title product **3c** as an off-white solid (166.9 mg, 80%).

¹H NMR (500 MHz, CDCl₃) δ 8.65 - 8.59 (m, 1H), 7.75 (dt, *J* = 7.0, 1.2 Hz, 1H), 7.44 (td, *J=* 7.6, 1.8 Hz, 1H), 7.36 - 7.31 (m, 1H), 7.31 - 7.23 (m, 2H), 7.12 - 7.01 (m, 4H), 6.90 - 6.80 (d, *J=* 8.9 Hz, 1H), 6.65 (m, 2H), 6.57 - 6.51 (m, 1H), 6.50 - 6.43 (m, 1H), 4.07 (t, *J* = 7.0 Hz, 2H), 2.92 (s, 4H), 2.55 - 2.40 (m, 6H), 2.17 (s, 3H), 2.04 (p, *J* = 7.1 Hz, 2H).

¹³C NMR (126 MHz, CDCl₃) δ 160.0, 150.5, 148.8, 148.7, 142.4, 141.8, 141.6, 139.3, 136.8, 136.0, 129.9, 129.4, 128.5, 128.1, 127.6, 125.7, 123.9, 121.4, 121.0, 118.2, 115.5, 114.1, 110.6, 55.7, 53.2, 49.0, 44.6, 26.2, 20.6

### Synthesis of [2-(o-tolyl)pyridine]-[Bupropion] (3d)

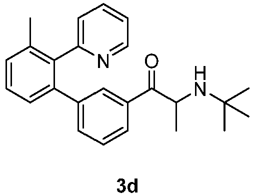

General procedure B was applied. 2-(*o*-tolyl)pyridine (67.8 mg, 0.4 mmol, 1 equiv), bupropion HCl (110.5 mg, 0.4 mmol, 1 equiv), KOAc (11.8 mg, 0.12 mmol, 30 mol %) and K₂CO₃ (165.8 mg, 1.2 mmol, 3 equiv) were reacted in NMP (0.8 mL) at 50 °C for 24 hours. After this time, quantitative ¹H NMR using 1,3,5-trimethoxy benzene as an internal standard indicated conversion to product 3d of 93%.

### Synthesis of [2-(o-tolyl)pyridine]-[chlormezanone] (3e)

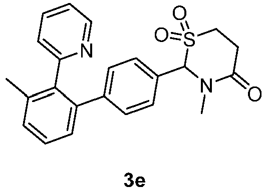

General procedure B was applied. 2-(*o*-tolyl)pyridine (67.8 mg, 0.4 mmol, 1 equiv), chlormezanone (109.0 mg, 0.4 mmol, 1 equiv), KOAc (11.8 mg, 0.12 mmol, 30 mol %) and K₂CO₃ (110.5 mg, 0.8 mmol, 2 equiv) were reacted in NMP (0.8 mL) at 50 °C for 24 hours. After this time, quantitative ¹H NMR using 1,3,5-trimethoxy benzene as internal standard indicated conversion to product **3e** of 90%.

### Synthesis of [2-(o-tolyl)pyridine]-[chlorpropham] (3f)

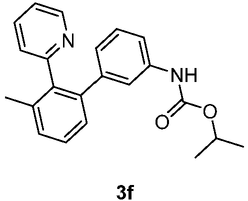

The General Procedure B was applied with 2-(*o-*tolyl)pyridine 1 (67.8 mg, 0.40 mmol, 1 equiv), Chlorpropham (85.4 mg, 0.40 mmol, 1 equiv) and K₂CO₃ (110.6 mg, 0.4 mmol, 2 equiv) allowing the reaction mixture to stir for 24 hours at 40 °C. Column chromatography eluting with 50% Et₂O in hexane afforded the title product **3f** as a white solid (117.7 mg, 85%).

¹H NMR (400 MHz, CDCl₃) δ 8.65 - 8.60 (m, 1H), 7.46 (td, *J* = 7.7, 1.8 Hz, 1H), 7.37 - 7.23 (m, 4H), 7.09 (ddd, *J* = 7.7, 4.9, 1.2 Hz, 1H), 7.06 - 6.99 (m, 2H), 6.91 (d, *J* = 7.8 Hz, 1H), 6.68 (d, *J* = 7.6 Hz, 1H), 6.36 (bs, 1H), 4.98 (hept, *J* = 6.2 Hz, 1H), 2.17 (s, 3H), 1.28 (d, *J* = 6.2 Hz, 6H).

¹³C NMR (126 MHz, CDCl₃) δ 159.6, 153.2, 149.0, 142.7, 140.9, 139.4, 137.7, 136.8, 135.9, 129.7, 128.4, 128.2, 127.7, 125.7, 125.0, 121.5, 119.8, 116.6, 68.8, 22.2, 20.6.

### Synthesis of [2-(o-tolyl)pyridine]-[δ-tocopherol] (3g)

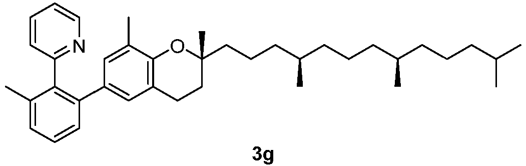

The General Procedure B was applied with 2-(*o*-tolyl)pyridine (33.9 mg, 0.20 mmol, 1 equiv), δ-Tocopherol-OTf (106.9 mg, 0.20 mmol, 1 equiv), [Ru(OH₂)(*t*BuCN)₅](BF₄)₂ **2** (14.2 mg, 0.02 mmol, 10 mol%) and K₂CO₃ (55.3 mg, 0.4 mmol, 2 equiv) allowing the reaction mixture to stir for 24 hours at 40 °C. Column chromatography eluting with 10% Et₂O in hexane afforded the title product **3g** as a colourless oil (86.4 mg, 90%).

¹H NMR (400 MHz, CDCl₃) δ 8.64 (d, *J* = 4.6 Hz, 1H), 7.46 (td, *J* = 7.7, 2.0 Hz, 1H), 7.35 - 7.27 (m, 2H), 7.23 (d, *J* = 7.2 Hz, 1H), 7.08 (dd, *J* = 8.0, 4.3 Hz, 1H), 6.89 (d, *J* = 7.7 Hz, 1H), 6.66 (s, 1H), 6.55 (s, 1H), 2.58 - 2.47 (m, 2H), 2.17 (s, 3H), 1.96 (s, 3H), 1.60-1.76 (m, 2H), 1.59 - 0.97 (m, 24H), 0.80-0.91 (m, 12H).

¹³C NMR (101 MHz, CDCl₃) δ 160.5, 151.0, 148.9, 141.6, 139.6, 136.8, 135.9, 132.4, 130.1, 129.0, 128.6, 128.2, 127.8, 126.0, 125.5, 121.3, 119.9, 76.3, 40.4, 39.7, 37.8, 37.6, 33.1, 33.0, 31.6, 28.3, 25.1, 24.8, 24.4, 23.1, 23.0, 22.5, 21.3, 20.9, 20.1, 20.0, 16.2.

### Synthesis of [2-(o-tolyl)pyridine]-[Haloperidol] (3h)

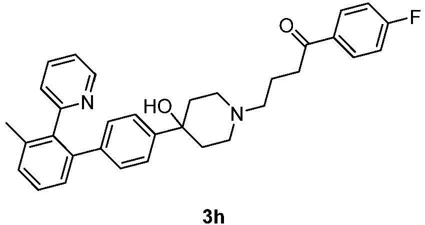

General procedure B was applied. 2-(*o*-tolyl)pyridine (67.8 mg, 0.4 mmol, 1 equiv), haloperidol (149.6 mg, 0.4 mmol, 1 equiv), KOAc (11.8 mg, 0.12 mmol, 30 mol %) and K₂CO₃ (110.5 mg, 0.8 mmol, 2 equiv) were reacted in NMP (0.8 mL) at 50 °C for 24 hours. After this time, quantitative ¹H NMR using 1,3,5-trimethoxy benzene as an internal standard indicated conversion to product **3h** of 87%.

### Synthesis of [2-(o-tolyl)pyridine]-[fenofibrate] (3i)

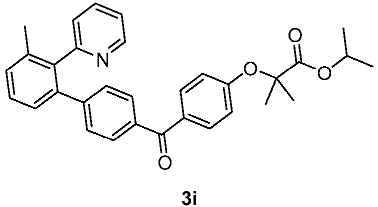

General procedure B was applied. 2-(*o*-tolyl)pyridine (67.8 mg, 0.4 mmol, 1 equiv), fenofibrate (144.4 mg, 0.4 mmol, 1 equiv), KOAc (11.8 mg, 0.12 mmol, 30 mol %) and K₂CO₃ (110.5 mg, 0.8 mmol, 2 equiv) were reacted in NMP (0.8 mL) at 40 °C for 24 hours. After this time, quantitative ¹H NMR using 1,3,5-trimethoxy benzene as internal standard indicated conversion to product **3i** of >99%.

### Synthesis of [2-(o-tolyl)pyridine]-[diazoxide] (3j)

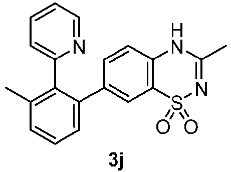

The General Procedure B was applied with 2-(*o*-tolyl)pyridine (97.8 mg, 0.40 mmol, 1 equiv), Diazoxide (92.2 mg, 0.40 mmol, 1 equiv) and K₂CO₃ (110.6 mg, 0.8 mmol, 2 equiv) allowing the reaction mixture to stir at 40 °C for 48 hours. After this time, quantitative ¹H NMR using 1,3,5-trimethoxy benzene as internal standard indicated conversion to product **3j** of 87%.

### Synthesis of [2-(o-tolyl)pyridine]-[meclizine] (3k)

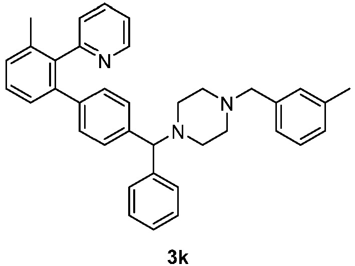

The General Procedure B was applied with 2-(o-tolyl)pyridine (67.8 mg, 0.40 mmol, 1 equiv), Meclizine • 2HCl (195.6 mg, 0.40 mmol, 1 equiv) and K₂CO₃ (221.2 mg, 1.6 mmol, 4 equiv) at 50 °C for 24 hours. After this time, quantitative ¹H NMR using 1,3,5-trimethoxy benzene as internal standard indicated conversion to product **3k** of >99%.

### Synthesis of [diazepam]-[5-m-xylene] (3l)

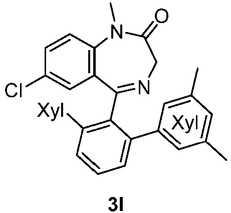

The General Procedure B was applied with diazepam (57.0 mg, 0.2 mmol, 1 equiv), I-*m*-xylene (58 µL mg, 0.40 mmol, 2 equiv) and K₂CO₃ (82.9 mg, 0.6 mmol, 3 equiv) allowing the reaction mixture to stir at 40 °C for 72 hours. After this time, quantitative ¹H NMR using 1,3,5-trimethoxy benzene as internal standard indicated conversion to product **3l** of 95%.

### Synthesis of 2-(3,3',5'-trimethyl-[1,1'-biphenyl]-2-yl)pyrimidine (3m)

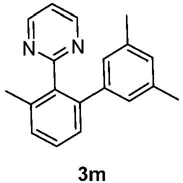

General procedure B was applied setting the reaction up in a microwave vial inside an argon filled glovebox. 2-(*o*-tolyl)pyrimidine (67.8 mg, 0.4 mmol, 1 equiv), br-*m*-xylene (74.0 mg, 0.4 mmol, 1 equiv), [Ru(OH₂)(*tBuCN*)₅](BF₄)₂ **2** (14.2 mg, 0.02 mmol, 5 mol %), KOAc (11.8 mg, 0.12 mmol, 30 mol %) and K₂CO₃ (110.5 mg, 0.8 mmol, 2 equiv) were reacted in NMP (0.8 mL) at 40 °C for 5 hours. After this time, quantitative ¹H NMR using 1,3,5-trimethoxy benzene as internal standard indicated conversion to product **3m** of 97%.

### Synthesis of 2-(3,3",5,5"-tetramethyl-[1,1':3',1"-terphenyl]-2'-yl)benzo[d]thiazole (3n)

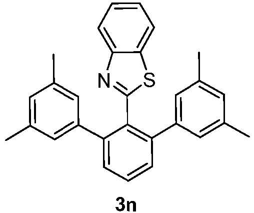

General procedure B was applied setting the reaction up in a microwave vial inside an argon filled glovebox. 2-phenylbenzo[d]thiazole (42.2 mg, 0.2 mmol, 1 equiv), br-*m*-xylene (74.0 mg, 0.4 mmol, 1 equiv), [Ru(OH₂)(*tBuCN*)₅](BF₄)₂ **2** (7.1 mg, 0.01 mmol, 5 mol %), KOAc (5.9 mg, 0.06 mmol, 30 mol %) and K₂CO₃ (55.3 mg, 0.4 mmol, 2 equiv) were reacted in NMP (0.4 mL) at 40 °C for 16 hours. After this time, quantitative ¹H NMR using 1,3,5-trimethoxy benzene as internal standard indicated conversion to product **3n** of 75%.

### Synthesis of 1-(3,3',5'-trimethyl-[1,1'-biphenyl]-2-yl)-1H-pyrazole (3o)

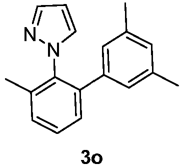

General procedure B was applied setting the reaction up in a microwave vial inside an argon filled glovebox. 1-(*o*-tolyl)-1H-pyrazole (31.6 mg, 0.2 mmol, 1 equiv), br-*m*-xylene (37.0 mg, 0.2 mmol, 1 equiv), [Ru(OH₂)(*tBuCN*)₅](BF₄)₂ **2** (7.1 mg, 0.01 mmol, 5 mol %), KOAc (5.9 mg, 0.06 mmol, 30 mol %) and K₂CO₃ (55.3 mg, 0.4 mmol, 2 equiv) were reacted in NMP (0.4 mL) at 40 °C for 16 hours. After this time, quantitative ¹H NMR using 1,3,5-trimethoxy benzene as internal standard indicated conversion to product **3o** of >99%.

### Synthesis of 2-(3',5'-dimethyl-[1,1'-biphenyl]-2-yl)-3-methylpyridine (3p)

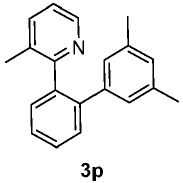

General procedure B was applied setting the reaction up in a microwave vial inside an argon filled glovebox. 3-methyl-2-phenylpyridine (67.8 mg, 0.4 mmol, 1 equiv), br-*m*-xylene (73.2 mg, 0.4 mmol, 1 equiv), [Ru(OH₂)(*tBuCN*)₅](BF₄)₂ **2** (14.2 mg, 0.02 mmol, 5 mol %), KOAc (11.8 mg, 0.12 mmol, 30 mol %) and K₂CO₃ (110.5 mg, 0.8 mmol, 2 equiv) were reacted in NMP (0.8 mL) at 40 °C for 5 hours. After this time, quantitative ¹H NMR using 1,3,5-trimethoxy benzene as internal standard indicated conversion to product **3p** of 40%.

### Synthesis of 3',5'-dimethyl-6-(pyridin-2-yl)-[1,1'-biphenyl]-3-ol (3q)

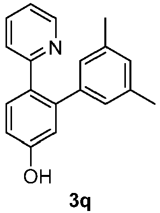

General procedure B was applied setting the reaction up in a microwave vial inside an argon filled glovebox. 4-(pyridin-2-yl)phenol (68.5 mg, 0.4 mmol, 1 equiv), 1-bromo-3,5-dimethylbenzene (146.4 mg, 0.4 mmol, 2 equiv), [Ru(OH₂)(*tBuCN*)₅](BF₄)₂ (**2**, 14.2 mg, 0.02 mmol, 5 mol%), KOAc (11.8 mg, 0.12 mmol, 30 mol %) and K₂CO₃ (110.5 mg, 0.8 mmol, 2 equiv) were reacted in NMP (0.8 mL) at 40 °C for 5 hours. After this time, quantitative ¹H NMR using 1,3,5-trimethoxy benzene as internal standard indicated conversion to product **3q** of 63%.

### General Procedure C: Ru-catalysed secondary alkylation of DG-containing arenes with secondary alkyl bromides

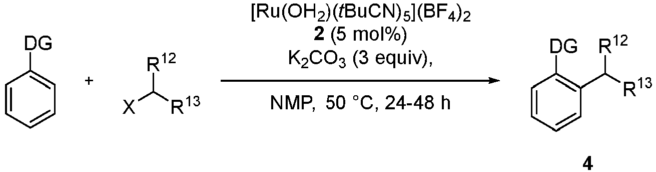

R¹² and R¹³ are substituents which may be linked to form a ring.

### General procedure for the secondary alkylation of DG-containing arenes

All liquid reagents were degassed with at least 3 freeze-pump-thaw cycles prior to use. A 10 mL Schlenk tube equipped with a magnetic stirring bar was charged with ruthenium aqua catalyst **2** (14.2 mg, 0.02 mmol, 5 mol%), K₂CO₃ (3.0 equiv), the appropriate DG-containing arene (0.40 mmol, 1 equiv) and the appropriate alkyl halide (1-2 equiv). After addition of the solids, 3 x 5 minute evac-refill cycles were performed before adding any liquid/oil reagents via injection along with the NMP (2 mL, 0.2 M with respect to the DG-containing arene). The vial was then stirred at 50 °C for the indicated time. The reaction was then allowed to cool to room temperature before being quenched with H₂O (20 mL) and extracted with Et₂O (3 × 20 mL). The organic extracts were combined, washed with brine (15 mL), dried over MgSO₄, filtered and concentrated in vacuo. The residue was purified by column chromatography under the conditions noted to yield the desired product.

Reactions were performed as set out below following General Procedure C.

Data for all compounds following this procedure matches that previously reported by Larrosa *et. al.*

G.-W. Wang, M. Wheatley, M. Simonetti, D. M. Cannas and I. Larrosa, Chem, 2020, 6, 1459-1468. 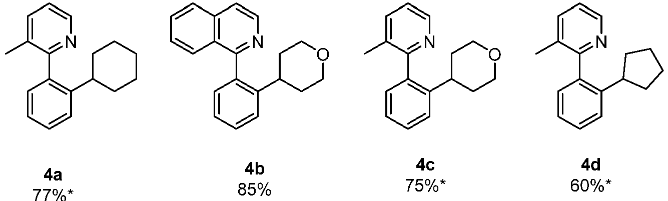 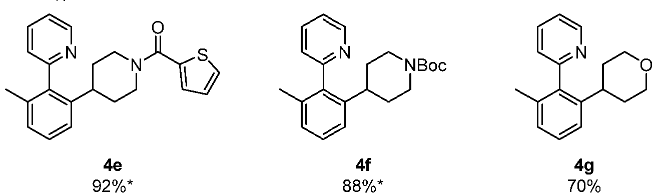 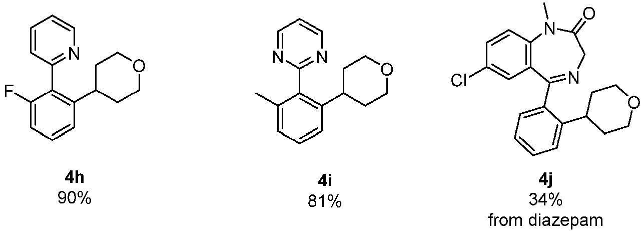 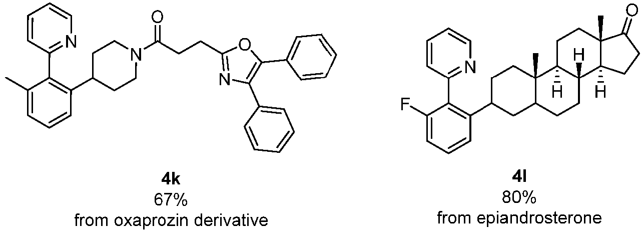 ** NMR yield calculated by ¹H Q NMR using trimethoxy benzene as internal standard.*

### Synthesis of 2-(2-cyclohexylphenyl)-3-methylpyridine (4a)

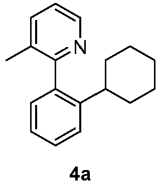

Obtained using General procedure C employing 3-methyl-2-phenylpyridine (67.8 mg, 0.40 mmol) and bromocyclohexane (73.8 µL, 0.60 mmol). The reaction was stirred at 50 °C for 24 hours. After this time, quantitative ¹H NMR using 1,3,5-trimethoxy benzene as internal standard indicated conversion to product **4a** of 77%.

### Synthesis of 1-(2-(tetrahydro-2H-pyran-4-yl)phenyl)isoquinoline (4b)

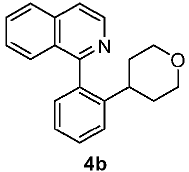

Obtained using General procedure C employing 1-phenylisoquinoline (94.4 mg, 0.40 mmol) and 4-bromotetrahydro-2H-pyran (47.4 µL, 0.42 mmol). The reaction was stirred at 50 °C for 24 hours. The crude mixture was purified by column chromatography eluting with 50% EtOAc in hexane to yield the title compound **4b** (98.4 mg, 89%) as a colourless oil.

¹H NMR (400 MHz, CDCl₃) δ 8.61 (d, *J* = 5.7 Hz, 1H), 7.91 (d, *J* = 8.3 Hz, 1H), 7.74 - 7.66 (m, 2H), 7.63 (d, *J* = 7.6 Hz, 1H), 7.55 - 7.40 (m, 3H), 7.42 - 7.14 (m, 1H), 7.28 (s, 1H), 3.95 - 3.87 (m, 1H), 3.88 - 3.77 (m, 1H), 3.15 (td, *J* = 11.4, 3.7 Hz, 1H), 3.03 - 2.93 (m, 1H), 2.50 (m, 1H), 1.90 - 1.71 (m, 3H), 1.41 - 1.33 (m, 1H).

¹³C NMR (101 MHz, CDCl₃) δ 161.2, 144.3, 142.1, 138.3, 136.3, 130.2, 129.9, 128.9, 128.0, 127.4, 127.2, 126.9, 126.4, 125.9, 120.0, 68.4, 68.2, 38.0, 34.1, 33.3.

### Synthesis of 3-methyl-2-(2-(tetrahydro-2H-pyran-4-yl)phenyl)pyridine (4c)

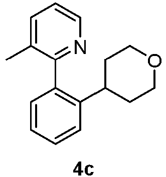

Obtained using General procedure C employing 3-methyl-2-phenylpyridine (67.8 mg, 0.40 mmol) and 4-bromotetrahydro-2H-pyran (47.4 µL, 0.42 mmol). The reaction was stirred at 50 °C for 24 hours. After this time, quantitative ¹H NMR using 1,3,5-trimethoxy benzene as internal standard indicated conversion to product **4c** of 75%.

### Synthesis of 2-(2-cyclopentylphenyl)-3-methylpyridine (4d)

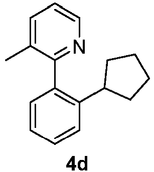

Obtained using General procedure C employing 3-methyl-2-phenylpyridine (67.8 mg, 0.40 mmol) and bromocyclopentane (64.0 µL, 0.6 mmol). The reaction was stirred at 50 °C for 24 hours. After this time, quantitative ¹H NMR using 1,3,5-trimethoxy benzene as internal standard indicated conversion to product **4d** of 60%.

### Synthesis of (4-(3-methyl-2-(pyridin-2-yl)phenyl)piperidin-1-yl)(thiophen-2-yl)methanone (4e)

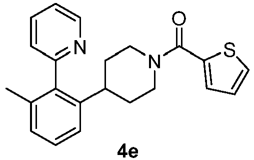

Obtained using General procedure C employing 2-(*o*-tolyl)pyridine (67.8 mg, 0.40 mmol) and (4-bromopiperidin-1-yl)(thiophen-2-yl)methanone (120.0 mg, 0.44 mmol. The reaction was stirred at 50 °C for 24 hours. After this time, quantitative ¹H NMR using 1,3,5-trimethoxy benzene as internal standard indicated conversion to product **4e** of 92%.

### Synthesis of tert-butyl 4-(3-methyl-2-(pyridin-2-yl)phenyl)piperidine-1-carboxylate (4f)

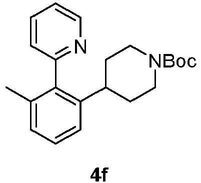

Obtained using General procedure C employing 2-(*o*-tolyl)pyridine (67.8 mg, 0.40 mmol) and (tert-butyl 4-bromopiperidine-1-carboxylate (132.0 mg, 0.50 mmol). The reaction was stirred at 50 °C for 24 hours. After this time, quantitative ¹H NMR using 1,3,5-trimethoxy benzene as internal standard indicated conversion to product **4f** of 92%.

### Synthesis of 2-(2-methyl-6-(tetrahydro-2H-pyran-4-yl)phenyl)pyridine (4g)

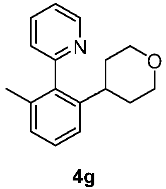

Obtained using General procedure C employing 2-(o-tolyl)pyridine (64.0 µL, 0.40 mmol) and 4-bromotetrahydro-2H-pyran (47.4 µL, 0.42 mmol). The reaction was stirred at 50 °C for 24 hours. After this time, quantitative ¹H NMR using 1,3,5-trimethoxy benzene as internal standard indicated conversion to product **4g** of 88%.

### Synthesis of 2-(2-fluoro-6-(tetrahydro-2H-pyran-4-yl)phenyl)pyridine (4h)

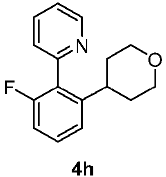

General Procedure C was applied. 2-(2-fluorophenyl)pyridine (69.2 mg, 0.40 mmol) and 4-bromotetrahydro-2H-pyran (47.4 µL, 0.42 mmol) were employed. The reaction was stirred at 50 °C for 24 hours. The crude mixture was purified by column chromatography eluting with 10% EtOAc in hexane to yield the title compound **4h** (98.0 mg, 90%) as a colourless oil.

¹H NMR (400 MHz, CDCl₃) δ 8.66 (d, *J* = 5.0 Hz, 1H), 7.70 (m, 1H), 7.34 - 7.15 (m, 3H), 7.11 (d, *J* = 7.9 Hz, 1H), 6.93 (m, 1H), 3.95-3.80 (m, 2H), 3.20-3.10 (t, *J* = 11.8 Hz, 2H), 2.75-2.60 (m, 1H), 1.80-1.65 (m, 2H), 1.63-1.50 (m, 2H).

¹³C NMR (101 MHz, CDCl₃) δ ¹³C NMR (101 MHz, CDCl₃) δ 160.2 (d, *J* = 245.5 Hz), 154.3, 149.8, 146.8 (d, *J* = 2.0 Hz), 136.4, 130.1 (d, *J* = 8.8 Hz), 128.2 (d, *J* = 15.6 Hz), 125.9 (d, *J*= 2.0 Hz), 122.7, 122.1 (d, *J* = 3.4 Hz), 113.5 (d, *J* = 22.5 Hz), 68.5, 37.7 (d, *J* = 2.4 Hz), 33.9.

¹⁹F NMR (376 MHz, CDCl₃) δ -115.42.

### Synthesis of 2-(2-methyl-6-(tetrahydro-2H-pyran-4-yl)phenyl)pyrimidine (4i)

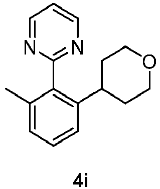

General Procedure C was applied. 2-(2-methyl-6-(tetrahydro-2H-pyran-4-yl)phenyl)pyrimidine (68.0 mg, 0.40 mmol and 4-bromotetrahydro-2H-pyran (47.4 µL, 0.42 mmol) were employed. The reaction was stirred at 50 °C for 18 hours. The crude mixture was purified by column chromatography eluting with 50% EtOAc in hexane to yield the title compound **2i** (82.3 mg, 81%) as a colourless solid.

¹H NMR (400 MHz, CDCl₃) δ 8.83 (d, *J* = 5.0 Hz, 2H), 7.31 - 7.22 (m, 2H), 7.19 (d, *J* = 7.7 Hz, 1H), 7.09 (d, *J=* 7.3 Hz, 1H), 4.09 - 3.78 (m, 2H), 3.32 - 3.00 (m, 2H), 2.31 (tt, *J=* 11.9, 3.8 Hz, 1H), 1.98 (s, 3H), 1.87 - 1.70 (m, 2H), 1.72 - 1.54 (m, 2H).

¹³C NMR (101 MHz, CDCl₃) δ 168.7, 157.3, 143.1, 138.8, 135.7, 129.0, 128.3, 123.8, 119.3, 68.6, 38.8, 33.9, 20.2.

### Synthesis of [diazepam]-[4-bromotetrahydro-2H-pyran] (4j)

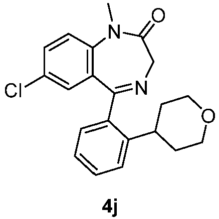

General Procedure C was applied. Diazepam (85.4 µL, 0.30 mmol), 4-bromotetrahydro-2H-pyran (74.2 mg, 0.45 mmol), K₂CO₃ (124.4 mg, 3 equiv) and [Ru(OH₂)(*tBuCN*)₅](BF₄)₂ **2** (14.2 mg, 0.02 mmol) were employed. The reaction was stirred in NMP (1 mL, 0.5 M with respect to the DG-containing arene) at 50 °C for 48 hours. The crude mixture was purified by column chromatography eluting with 65% EtOAc in hexane to yield the title compound **4j** (35.0 mg, 34%) as a colourless oil.

¹H NMR (500 MHz, CDCl₃) δ 7.44 - 7.34 (m, 2H), 7.31 - 7.15 (m, 4H), 6.97 (d, *J* = 2.6 Hz, 1H), 4.80 (d, *J* = 10.8 Hz, 1H), 3.97 - 3.67 (m, 3H), 3.42 (s, 3H), 3.11 (t, *J* = 11.7 Hz, 1H), 2.90 (t, *J* = 12.2 Hz, 1H), 2.33 (t, *J* = 12.1 Hz, 1H), 1.76 - 1.56 (m, 2H), 1.48 (d, *J* = 13.4 Hz, 1H), 1.06 (d, *J* = 13.4 Hz, 1H).

¹³C NMR (126 MHz, CDCl₃) δ 170.8, 169.8, 143.7, 141.3, 138.1, 132.1, 131.5, 130.1, 129.6, 129.4, 129.2, 126.8, 126.3, 122.3, 68.6, 68.3, 56.8, 38.4, 35.0, 33.9, 33.0.

### 3-(4,5-diphenyloxazol-2-yl)-1-(4-(3-methyl-2-(pyridin-2-yl)phenyl)piperidin-1-yl)propan-1-one (4k) from oxaprozin derivative

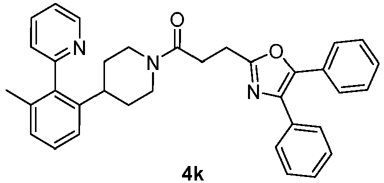

General Procedure C was applied. 2-(*o*-tolyl)pyridine (67.8 mg, 0.40 mmol) and 1-(4-bromopiperidin-1-yl)-3-(4,5-diphenyloxazol-2-yl)propan-1-one (194.0 mg, 0.44 mmol) were employed. The reaction was stirred at 50 °C for 48 hours. The crude mixture was purified by column chromatography eluting with 65% EtOAc in hexane to yield the title compound **4k** (146.0 67%mg, 67%) as a colourless oil.

¹H NMR (400 MHz, CDCl₃) δ 8.72 (d, *J* = 5.0 Hz, 1H), 7.78 - 7.73 (m, 1H), 7.63 - 7.58 (m, 2H), 7.57 - 7.52 (m, 2H), 7.39 - 7.27 (m, 7H), 7.25 (t, *J* = 7.7 Hz, 2H), 7.12 - 7.04 (m, 2H), 4.68 (d, *J* = 13.0 Hz, 1H), 3.96 - 3.87 (m, 1H), 3.34 - 3.09 (m, 2H), 3.04 - 2.68 (m, 3H), 2.49 - 2.21 (m, 2H), 2.00 (s, 3H), 1.93 - 1.75 (m, 1H), 1.75 - 1.46 (m, 3H).

¹³C NMR (101 MHz, CDCl₃) δ 169.3, 162.9, 159.7, 149.8, 145.4, 142.9, 140.0, 136.4, 136.2, 135.2, 132.7, 129.1, 128.7, 128.7, 128.5, 128.5, 128.2, 128.1, 128.1, 126.5, 124.7, 123.5, 122.0, 46.3, 42.7, 39.4, 33.8, 32.8, 30.1, 24.0, 20.6.

### Synthesis of [2-(2-fluorophenyl)pyridine]-[epiandrosterone] 4l

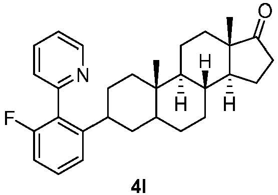

Obtained by using General procedure C. 1d (34.6 mg, 0.20 mmol), 3α-21 (106 mg, 0.30 mmol), K₂CO₃ (124.4 mg, 3 equiv) and [Ru(OH₂)(*tBuCN*)₅](BF₄)₂ **2** (14.2 mg, 0.02 mmol) were employed. The reaction was stirred at 50 °C for 24 hours. After this time, quantitative ¹H NMR using 1,3,5-trimethoxy benzene as internal standard indicated conversion to product **4l** of 80%.

### General Procedure D: Ru-catalysed secondary alkylation of DG-containing arenes with primary alkyl bromides

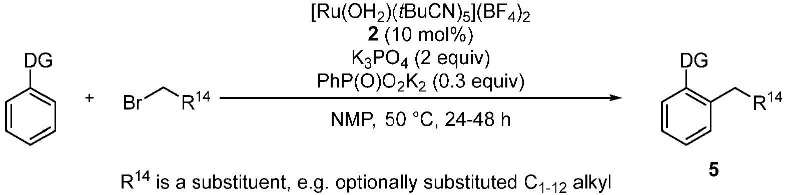

### General procedure for the primary alkylation of DG-containing arenes

All liquid reagents were degassed with at least 3 freeze-pump-thaw cycles prior to use. Tribasic potassium phosphate (K₃PO₄) and potassium phenylphosphonate (PhP(O)O₂K₂) were dried at 80 °C in a vacuum oven for at least 48 hours prior to use. Unless otherwise indicated, a 10 mL Schlenk tube was charged with ruthenium aqua catalyst **2** (28.4 mg, 0.04 mmol, 10 mol%), K₃PO₄ (254.7 mg, 1.2 mmol, 3 equiv), and potassium phenylphosphonate (29.0 mg, 0.12 mmol, 30 mol), the appropriate DG-containing arene (0.40 mmol, 1 equiv) and the appropriate alkyl halide (1-2 equiv). After addition of the solids, 3 x 5 minute evac-refill cycles were performed before adding any liquid/oil reagents via injection along with the NMP (2 mL, 0.2 M with respect to the DG-containing arene). The reaction mixture was then stirred at 50 °C for 24 hours. Upon completion, the reaction mixture was diluted with water and washed with Et₂O, before being dried over MgSO₄, filtered, and concentrated in vacuo. The crude mixture was then loaded onto a silica gel column and purified using flash chromatography.

Reactions were performed as set out below following General Procedure D.

Data for all compounds following this procedure matches that previously reported by Larrosa *et. al.*

M. Wheatley, M. T. Findlay, R. López-Rodríguez, D. M. Cannas, M. Simonetti and I. Larrosa, Chem Catalysis, 2021, 1, 691-703. 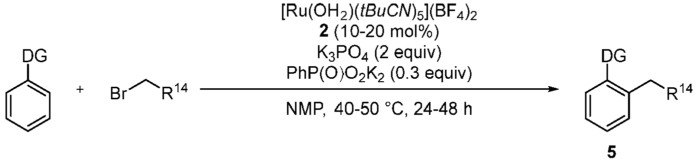 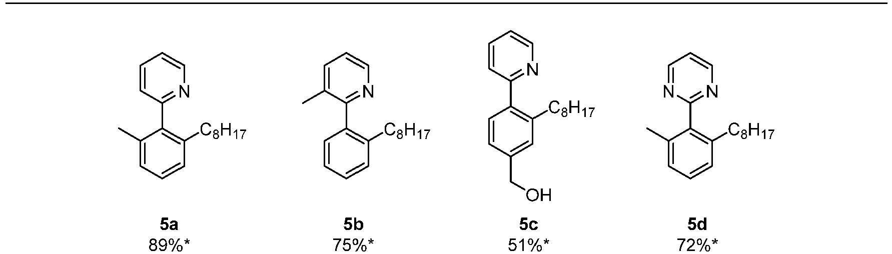 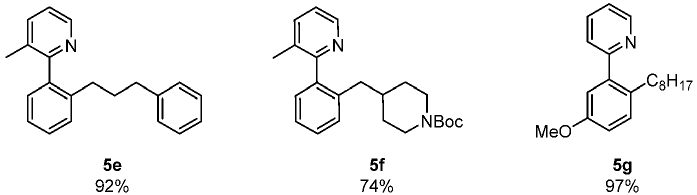 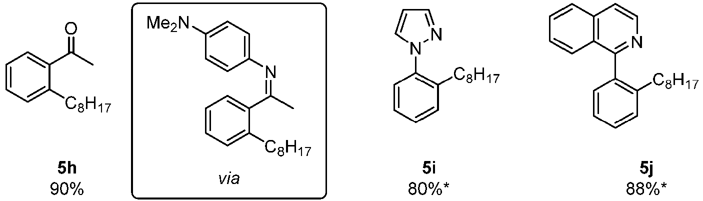 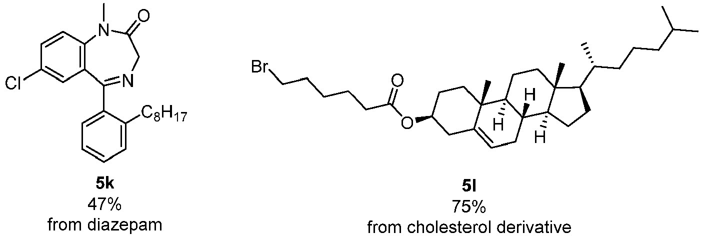 ** NMR yield calculated by ¹H Q NMR using trimethoxy benzene as internal standard.*

### Synthesis of 2-(2-methyl-6-octylphenyl)pyridine (5a)

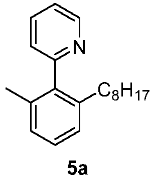

The reaction was carried out following General Procedure D with 2-(*o*-tolyl)pyridine (67.8 mg, 0.40 mmol) and 1-bromooctane (138.7.0 µL, 0.80 mmol) allowing the reaction mixture to stir for 72 hours. After this time, quantitative ¹H NMR using 1,3,5-trimethoxy benzene as internal standard indicated conversion to product **5a** of 89%.

### Synthesis of 3-methyl-2-(2-octylphenyl)pyridine (5b)

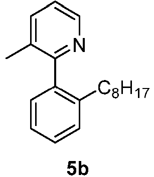

The reaction was carried out following General Procedure D with 3-methyl-2- phenylpyridine (67.8 mg, 0.40 mmol) and 1-bromooctane (138.7.0 µL, 0.80 mmol) stirring for 24 hours. After this time, quantitative ¹H NMR using 1,3,5-trimethoxy benzene as internal standard indicated conversion to product **5b** of 75%.

### Synthesis of (3-octyl-4-(pyridine-2-yl)phenyl)methanol (5c)

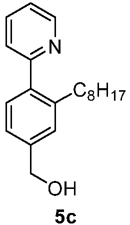

The reaction was carried out following General Procedure D. (4-(pyridine-2-yl)phenyl)methanol (74.1 mg, 0.40 mmol) and 1-bromooctane (138.7.0 µL, 0.80 mmol) were used allowing the reaction mixture to stir for 24 hours. After this time, quantitative ¹H NMR using 1,3,5-trimethoxy benzene as internal standard indicated conversion to product 5c of 51%.

### Synthesis of 2-(2-methyl-6-octylphenyl)pyrimidine (5d)

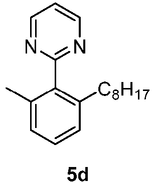

The reaction was carried out following General Procedure D. 2-phenylpyrimidine (68.1 mg, 0.40 mmol) and 1-bromooctane (138.7.0 µL, 0.80 mmol) were used allowing the reaction mixture to stir for 24 hours. After this time, quantitative ¹H NMR using 1,3,5-trimethoxy benzene as internal standard indicated conversion to product 5d of 72%.

### Synthesis of 3-methyl-2-(2-(3-phenylpropyl)phenyl)pyridine (5e)

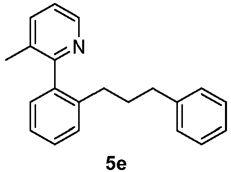

The reaction was carried out following General Procedure D, with the use of 3-methyl-2-phenylpyridine (67.8 mg, 0.40 mmol) and (3-bromopropyl)benzene (159.3 mg, 0.40 mmol). The crude reaction mixture was purified by column chromatography eluting with 0-10% EtOAc-DCM mixture (1:1) in hexane to give the named compound **5e** (105.8 mg, 92%) as a colourless oil.

¹H NMR (500 MHz, CDCl₃) δ 8.43 (d, *J* = 4.7 Hz, 1H), 7.47 (d, *J* = 7.8 Hz, 1H), 7.29 - 7.22 (m, 2H), 7.29 - 7.24 (m, 1H), 7.17 - 7.04 (m, 5H), 6.96 (d, *J* = 7.0 Hz, 2H), 2.50 - 2.45 (m, 4H), 2.02 (s, 3H), 1.77 - 1.61 (m, 2H).

¹³C NMR (126 MHz, CDCl₃) δ 159.5, 146.6, 142.2, 140.1, 140.0, 137.7, 131.5, 129.4, 128.9, 128.3, 128.2, 128.1, 125.9, 125.6, 122.2, 35.6, 32.6, 32.2, 19.3.

### Synthesis of tert-butyl 4-(2-(3-methylpyridin-2-yl)benzyl)piperidine-1-carboxylate (5f)

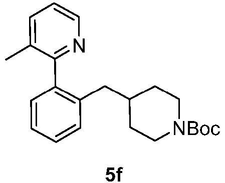

The reaction was carried out following General Procedure D, with the use of 3-methyl-2-phenylpyridine (67.8 mg, 0.40 mmol) and tert-butyl 4-(bromomethyl)piperidine-1-carboxylate (222.5 mg, 0.80 mmol). The crude reaction mixture was purified by column chromatography eluting with 0-10% EtOAc-DCM mixture (1:1) in hexane to give the named compound **5f** (108.5 mg, 74%) as a light yellow oil.

¹H NMR (400 MHz, CDCl₃) δ 8.42 (dd, *J* = 4.8, 1.8 Hz, 1H), 7.50 (d, *J* = 7.7 Hz, 1H), 7.27 - 7.14 (m, 3H), 7.14 - 7.06 (m, 2H), 3.87 (s, 2H), 2.57 - 2.16 (m, 4H), 2.01 (s, 3H), 1.49 - 1.30 (s, 12H), 0.84 (qd, *J* = 12.5, 4.4 Hz, 2H).

¹³C NMR (126 MHz, CDCl₃) δ 159.5, 146.6, 142.2, 140.0, 137.7, 131.5, 129.4, 128.9, 128.3, 128.2, 128.1, 125.9, 125.6, 122.2, 35.6, 32.6, 32.2, 19.3.

### Synthesis of 2-(5-methoxy-2-octylphenyl)pyridine (5g)

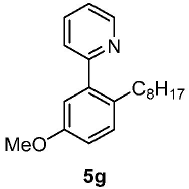

The reaction was carried out following General Procedure D, with the use of 2-(3-methoxyphenyl)pyridine (72.3 mg, 0.40 mmol) and 1-bromooctane (138.6 µL, 0.80 mmol). The crude reaction mixture was purified by column chromatography eluting with 0-10% EtOAc-DCM mixture (1:1) in hexane to give the named compound **5g** (50.0 mg, 56%) as a colourless oil.

¹H NMR (500 MHz, CDCl₃) δ 8.71 - 8.66 (m, 1H), 7.70 (app. Td, *J* = 7.6, 2.3 Hz, 1H), 7.30 - 7.18 (m, 3H), 6.91 - 6.87 (m, 1H), 6.79 (d, *J* = 8.2 Hz, 1H), 3.67 (s, 3H), 2.38 - 2.30 (m, 2H), 1.44 - 1.36 (m, 2H), 1.28 - 1.04 (m, 10H), 0.83 (t, *J* = 7.2 Hz, 3H).

¹³C NMR (126 MHz, CDCl₃) δ 157.1, 157.0, 149.3, 142.8, 135.7, 129.6, 128.9, 125.8, 121.7, 121.6, 108.3, 55.7, 33.0, 31.9, 31.0, 29.5, 29.2, 29.1, 22.7, 14.2.

### Synthesis of 1-(2-octylphenyl)ethan-1-one (5h)

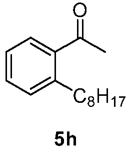

The reaction was carried out following General Procedure D, with the use of (E)-N,N-dimethyl-4-((1- phenylethylidene)amino)aniline (95.3 mg, 0.4 mmol) and 1-bromooctane (138.2 µL, 0.8 mmol) allowing the reaction mixture to stir for 72 hours. K₂CO₃ (110.5 mg, 2 equiv) was used as the base and KOAc (11.8 mg, 30 mol%) as the additive. After 72 hours, HCl (aq. 3M, 2 mL) was added to the reaction mixture, and stirred for a further 1 hour, before being washed with ether. The crude reaction mixture was purified by column chromatography eluting with 0-5% EtOAc in hexane to give the named product **5h** as a colourless oil (84.0 mg, 90%).

¹H NMR (500 MHz, CDCl₃) δ 7.61 (dd, *J=* 8.3, 1.3 Hz, 1H), 7.38 (td, *J* = 7.2, 1.4 Hz, 1H), 7.28 - 7.21 (m, 2H), 2.87 - 2.79 (m, 2H), 2.57 (s, 3H), 1.61 - 1.50 (m, 2H), 1.40 - 1.21 (m, 10H), 0.94 - 0.80 (m, 3H).

¹³C NMR (101 MHz, CDCl₃) δ 202.3, 142.9, 138.1, 131.2, 131.1, 128.9, 125.6, 34.0, 31.9, 30.0, 29.8, 29.5, 29.3, 27.2, 22.7, 14.1.

### Synthesis of 1-(2-octylphenyl)-1H-pyrazole 5i

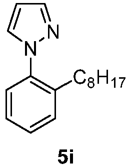

The reaction was carried out following General Procedure D, with the use of 1-phenyl-1H-pyrazole (57.7 mg, 0.4 mmol) and 1-bromooctane (138.2 µL, 0.8 mmol) allowing the reaction to stir for 24 hours. After this time, quantitative ¹H NMR using 1,3,5-trimethoxy benzene as internal standard indicated conversion to product **5i** of 80%.

### Synthesis of 1-(2-octylphenyl)isoquinoline (5j)

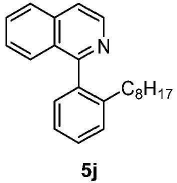

The reaction was carried out following General Procedure D, with the use of 1-phenylisoquinoline (82.1 mg, 0.4 mmol) and 1-bromooctane (138.2 µL, 0.8 mmol) allowing the reaction to stir for 24 hours. After this time, quantitative ¹H NMR using 1,3,5-trimethoxy benzene as internal standard indicated conversion to product **5j** of 88%.

### Synthesis of [diazepam]-[n-octyl] (5k)

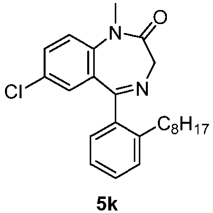

The reaction was carried out following General Procedure D, with the use of diazepam (57.0 mg, 0.2 mmol), K₃PO₄ (127.4 mg, 2 equiv), PhP(O)O₂K₂ (14.3 mg, 30 mol%), [Ru(OH₂)(*t*BuCN)₅](BF₄)₂ **2** (14.2 mg, 0.02 mmol, 10 mol%) and 1-bromooctane (35.0 µL, 0.4 mmol). The crude reaction mixture was purified by column chromatography eluting with 0-30% EtOAc in hexane to give the named product **5k** (37.1 mg, 47%) as a colourless oil.

¹H NMR (500 MHz, CDCl₃) δ 7.47 - 7.42 (m, 1H), 7.37 - 7.30(m, 2H), 7.25 (d, *J* = 8.7 Hz, 2H), 7.18 (d, *J* = 7.8 Hz, 1H), 7.03 (d, *J* = 2.7 Hz, 1H), 4.83 (d, *J* = 10.8 Hz, 1H), 3.78 (d, *J* = 10.8 Hz, 1H), 3.41 (s, 3H), 2.37 - 2.26 (m, 1H), 2.20 - 2.06 (m, 1H), 1.38 - 0.92 (m, 12H), 0.83 (t, *J* = 7.1 Hz, 3H).

¹³C NMR (126 MHz, CDCl₃) δ 171.1, 169.8, 141.64, 141.3, 138.5, 132.0, 131.5, 130.1, 130.0, 129.9, 129.6, 129.3, 126.1, 122.5, 56.9, 35.0, 33.6, 31.9, 31.1, 29.9, 29.5, 29.3, 22.8, 14.2.

### (3S,8S,9S,10R,13R,14S,17R)-10,13-dimethyl-17-((R)-6-methylheptan-2-yl)-2,3,4,7,8,9,10,11,12,13,14,15,16,17-tetradecahydro-1H-cyclopenta[a]phenanthren-3-yl 6-(2-(3-methylpyridin-2-yl)phenyl)hexanoate (5l) from cholesterol derivative

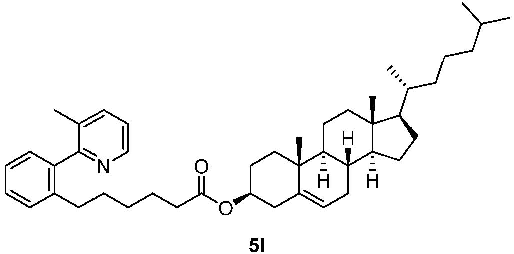

The reaction was carried out following General Procedure D, with the use of 3-methyl-2-phenylpyridine (33.9 mg, 0.2 mmol), (3S,8S,9S,10R,13R,14S,17R)-10,13-dimethyl-17-((R)-6-methylheptan-2-yl)-2,3,4,7,8,9,10,11,12,13,14,15,16,17-tetradecahydro-1Hcyclopentala]phenanthren-3-yl 2-bromoacetate (225.5, 0.40 mmol), K₃PO₄ (127.4 mg, 2 equiv), PhP(O)O₂K₂ (14.3 mg, 30 mol%) and [Ru(OH₂)(*t*BuCN)₅](BF₄)₂ **2** (14.2 mg, 0.02 mmol, 10 mol%). The crude reaction mixture was purified by column chromatography eluting with 0-10% EtOAc-DCM mixture (1:1) in hexane to give the named compound **5l** (97.8 mg, 75%) as a light brown oil.

¹H NMR (400 MHz, CDCl₃) δ 8.46 (dd, *J* = 4.8, 1.9 Hz, 1H), 7.54 (dd, *J* = 7.7, 1.8 Hz, 1H), 7.31 - 7.18 (m, 3H), 7.15 (dd, *J* = 7.7, 4.8 Hz, 1H), 7.13 - 7.08 (m, 1H), 5.33 (d, *J* = 4.9 Hz, 1H), 4.63 - 4.52 (m, 1H), 2.49 - 2.29 (m, 2H), 2.24 (d, *J* = 8.3 Hz, 2H), 2.12 (t, *J* = 7.6 Hz, 2H), 2.07 (s, 3H), 2.02 - 1.88 (m, 2H), 1.86 - 1.73 (m, 3H), 1.59 - 1.02 (m, 24H), 1.02 - 0.90 (m, 6H), 0.88 (d, *J* = 6.6 Hz, 3H), 0.83 (dd, *J* = 6.6, 1.8 Hz, 6H), 0.65 (s, 3H).

¹³C NMR (101 MHz, CDCl₃) δ 173.2, 159.7, 146.7, 140.2, 140.0, 139.8, 137.8, 131.6, 129.4, 128.8, 128.1, 125.8, 122.7, 122.3, 73.7, 56.8, 56.2, 50.1, 42.4, 39.8, 39.6, 38.3, 37.1, 36.7, 36.3, 35.9, 34.6, 32.8, 32.0, 32.0, 30.3, 28.9, 28.4, 28.1, 27.9, 24.8, 24.4, 23.9, 22.9, 22.7, 21.1, 19.4, 19.4, 18.8, 12.0.

### General Procedure E: Ru-catalysed methylation of DG-containing arenes with ammonium salt

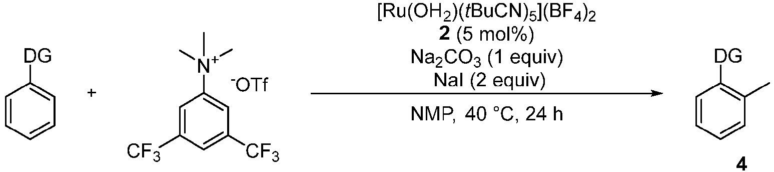

All liquid reagents were degassed with at least 3 freeze-pump-thaw cycles prior to use. To an oven-dried 10 mL Schlenk tube containing a magnetic stirring bar was added ruthenium aqua catalyst **2** (5 mol%, 0.015 mmol, 10.6 mg), NaI (89.9 mg, 2 equiv., 0.6 mmol), the methylating ammonium salt (1 equiv., 0.3 mmol, 56.0 mg), Na₂CO₃ (31.8 mg, 1 equiv., 0.3 mmol,) and the DG-containing arene (1 equiv., 0.3 mmol) before performing 3 x 5 minute evac-refill cycles. NMP (1.5 mL, 0.2 M with respect to the DG-containing arene) was then added. The reaction mixture was stirred at 40 °C for 24 hours. Upon completion, the crude reaction mixture was diluted with water (30 mL) and extracted with Et₂O (3 x 30 mL) before being loaded onto a silica gel column and purified by flash column chromatography under the conditions noted to afford pure product.

The following reactions were performed following General Procedure E:

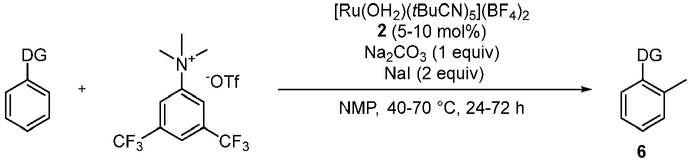

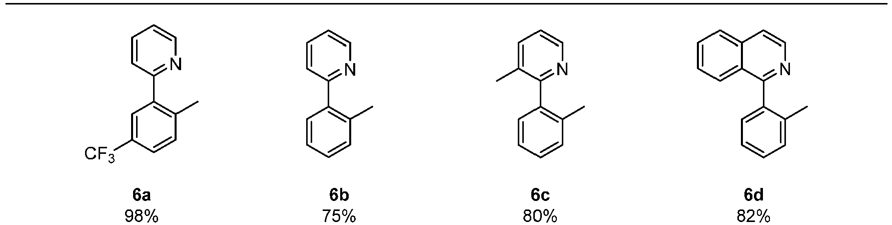

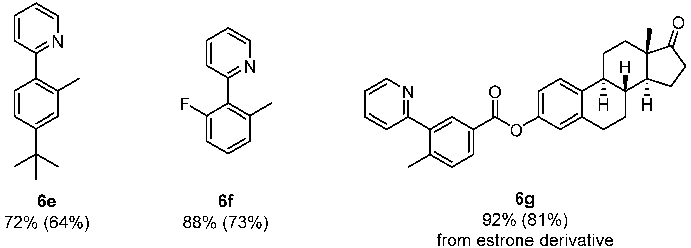

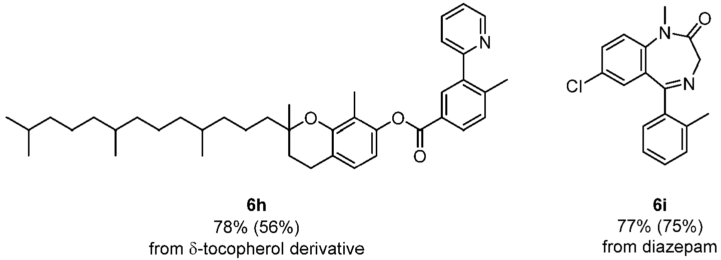

*Yields were calculated using ¹H QNMR with nitromethane as an internal standard. Isolated yields are given in brackets.*

### Synthesis of 2-(2-methyl-5-(trifluoromethyl)phenyl)pyridine (6a)

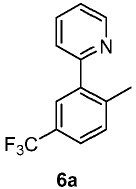

The title compound was synthesised as outlined in General Procedure E, using 2-(3-(trifluoromethyl)phenyl)pyridine (71.1 mg, 0.3 mmol) and [Ru(OH₂)(*t*BuCN)₅](BF₄)₂ **2** (10.6 mg, 0.015 mmol) for 24 hours. After this time, quantitative ¹H NMR using nitromethane as internal standard indicated conversion to product **6a** of 98%.

### Synthesis of 2-(o-tolyl)pyridine (6b)

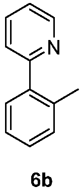

The title compound was synthesised as outlined in General Procedure E, using 2-phenylpyridine (46.5 mg, 0.3 mmol) and [Ru(OH₂)(*t*BuCN)₅](BF₄)₂ **2** (10.6 mg, 0.015 mmol) for 24 hours. After this time, quantitative ¹H NMR using nitromethane as internal standard indicated conversion to product **6b** of 75%.

### Synthesis of 3-methyl-2-(o-tolyl)pyridine (6c)

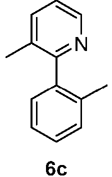

The title compound was synthesised as outlined in General Procedure E, using 3-methyl-2-phenylpyridine (50.7 mg, 0.3 mmol) and [Ru(OH₂)(*t*BuCN)₅](BF₄)₂ **2** (10.6 mg, 0.015 mmol) for 24 hours. After this time, quantitative ¹H NMR using nitromethane as internal standard indicated conversion to product **6c** of 80%.

### Synthesis of 1-(o-tolyl)isoquinoline (6d)

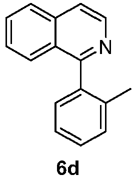

The title compound was synthesised as outlined in General Procedure E, using 1-phenylisoquinolie (61.5 mg, 0.3 mmol) and [Ru(OH₂)(*t*BuCN)₅](BF₄)₂ **2** (10.6 mg, 0.015 mmol) for 24 hours. After this time, quantitative ¹H NMR using nitromethane as internal standard indicated conversion to product **6d** of 82%.

### Synthesis of 2-(4-(tert-butyl)-2-methylphenyl)pyridine (6e)

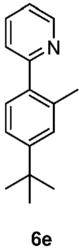

The title compound was synthesised as outlined in General Procedure E, using 2-(4-(*tert-*butyl)phenyl)pyridine (63 mg, 0.3 mmol) and [Ru(OH₂)(*tBuCN*)₅](BF₄)₂ **2** (10.6 mg, 0.015 mmol, 5 mol%). The reaction was allowed to stir for 24 hours. After this time, quantitative ¹H NMR using nitromethane as internal standard indicated conversion to product **6e** of 72%. Purification using flash column chromatography eluting with 0-10% EtOAc in hexane afforded the named compound **6e** as a yellow oil (43.2 mg, 64 %).

¹H NMR (400 MHz, CDCl₃) δ 8.59 - 8.51 (m, 1H), 7.59 (td, *J =* 7.7, 1.8 Hz, 1H), 7.27 (m, 1H), 7.22 (d, *J* = 8.7 Hz, 1H), 7.17 (d, *J* = 2.8 Hz, 2H), 7.08 (m, 1H), 2.25 (s, 3H), 1.22 (s, 9H).

¹³C NMR (101 MHz, CDCl₃) δ 160.1, 151.2, 149.2, 137.7, 136.0, 135.2, 129.4, 127.8, 124.0, 122.9, 121.4, 34.5, 31.4, 20.6.

### Synthesis of 2-(2-fluoro-6-methylphenyl)pyridine (6f)

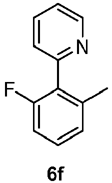

The title compound was synthesised as outlined in General Procedure E, using 2-(2-fluorophenyl)pyridine (52 mg, 0.3 mmol and [Ru(OH₂)(*t*BuCN)₅](BF₄)₂ **2** (10.6 mg, 0.015 mmol, 5 mol%). The reaction was allowed to stir for 24 hours. After this time, quantitative ¹H NMR using nitromethane as internal standard indicated conversion to product **6f** of 88%. Purification using flash column chromatography eluting with 0-10% EtOAc in hexane afforded the named compound **6f** as a yellow oil (33.7 mg, 73 %)
¹H NMR (400 MHz, CDCl₃) δ 8.73 (m, 1H), 7.76 (td, *J* = 7.7, 1.8 Hz, 1H), 7.40 - 7.32 (m, 1H), 7.32 - 7.19 (m, 2H), 7.07 (d, *J =* 7.6 Hz, 1H), 6.98 (t, *J =* 8.8 Hz, 1H), 2.21 (s, 3H).

¹³C NMR (101 MHz, CDCl₃) δ 160.2 (d, *J =* 245.0 Hz), 154.4, 149.6, 139.0 (d, *J =* 2.4 Hz), 136.2, 129.4 (d, *J =* 8.8 Hz), 128.4 (d, *J =* 15.2 Hz), 125.9 (d, *J =* 3.4 Hz), 125.5 (d, *J =* 2.4 Hz), 122.3, 113.0 (d, *J* = 22.5 Hz), 19.74 (d, *J =* 2.4 Hz).

¹⁹F NMR (376 MHz, CDCl₃) δ -117.15.

### (8R,9S,13S,14S)-13-methyl-17-oxo-7,8,9,11,12,13,14,15,16,17-decahydro-6H-cyclopenta[a]phenanthren-3-yl 4-methyl-3-(pyridin-2-yl)benzoate (6g) from estrone derivative

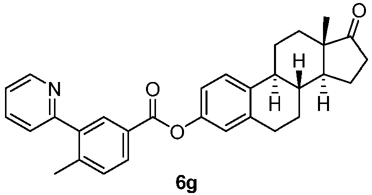

The title compound was synthesised as outlined in General Procedure E, estrone derivative (135.5 mg, 0.3 mmol) and [Ru(OH₂)(*t*BuCN)₅](BF₄)₂ **2** (10.6 mg, 0.015 mmol, 5 mol%) were employed. The reaction was allowed to stir for 24 hours. After this time, quantitative ¹H NMR using nitromethane as internal standard indicated conversion to product **6g** of 92%. Purification using flash column chromatography eluting with 15-25% EtOAc in hexane afforded the named compound **6g** as a colourless oil (113.0 mg, 81%).

¹H NMR (400 MHz, CDCl₃) δ 8.74 - 8.71 (m, 1H), 8.22 (d, *J* = 2.0 Hz, 1H), 8.11 (dd,*J* = 7.9, 2.0 Hz, 1H), 7.79 (td, *J* = 7.7, 1.8 Hz, 1H), 7.46 (dt, *J* = 7.8, 1.1 Hz, 1H), 7.42 (d, *J* = 7.9 Hz, 1H), 7.35 - 7.27 (m, 2H), 6.98 (dd, *J =* 8.8, 2.9 Hz, 1H), 6.94 (d, *J =* 2.6 Hz, 1H), 2.97 - 2.89 (m, 2H), 2.56 - 2.39 (m, 5H), 2.37 - 2.27 (m, 1H), 2.21- 1.94 (m, 4H), 1.70 -1.41 (m, 6H), 0.93 (s, 3H).

¹³C NMR (101 MHz, CDCl₃) δ 220.8, 165.3, 159.0, 149.4, 148.9, 142.3, 140.8, 138.1, 137.4, 136.4, 131.4, 131.2, 129.9, 127.4, 126.5, 124.3, 122.1, 121.8, 118.9, 50.5, 48.0, 44.2, 38.0, 35.9, 31.6, 29.5, 26.4, 25.8, 21.6, 20.7, 13.9.

### 2,8-dimethyl-2-(4,8,12-trimethyltridecyl)chroman-7-yl-4-methyl-3-(pyridin-2-yl)benzoate (6h) from δ-tocopherol derivative

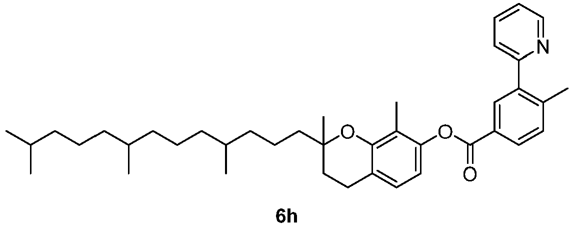

The title compound was synthesised as outlined in General Procedure E, using the δ-tocopherol-DG-containing-derivative-arene (175.2 mg, 0.3 mmol) and [Ru(OH₂)(*t*BuCN)₅](BF₄)₂ **2** (10.6 mg, 0.015 mmol, 5 mol%). The reaction was allowed to stir for 24 hours. After this time, quantitative ¹H NMR using nitromethane as internal standard indicated conversion to product **6h** of 78%. Purification using flash column chromatography eluting with 0-10% EtOAc in hexane afforded the named compound as a colourless oil (90.7 mg, 56%).

¹H NMR (400 MHz, CDCl3) δ 8.75 - 8.71 (m, 1H), 8.22 (d, J = 1.9 Hz, 1H), 8.11 (dd, J = 7.9, 1.9 Hz, 1H), 7.78 (td, J = 7.7, 1.8 Hz, 1H), 7.45 (dt, J = 7.8, 1.1 Hz, 1H), 7.41 (d, J = 8.0 Hz, 1H), 7.28 (ddd, J = 7.5, 4.9, 1.1 Hz, 1H), 6.81 (dd, J = 2.8, 0.9 Hz, 1H), 6.75 (d, J = 2.8 Hz, 1H), 2.82 - 2.69 (m, 2H), 2.46 (s, 3H), 2.18 (s, 3H), 1.89 - 1.69 (m, 2H), 1.67 - 0.99 (m, 24H), 0.87 (m, 12H).

¹³C NMR (101 MHz, CDCl3) δ 166.0, 159.3, 150.1, 149.6, 143.0, 142.4, 141.0, 136.7, 131.6, 131.4, 130.1, 128.0, 127.6, 124.4, 122.4, 121.6, 121.3, 119.5, 76.4, 40.4, 39.7, 37.8, 37.7 (2C), 37.6, 33.1, 33.0, 31.3, 28.3, 25.1, 24.8, 24.5, 23.0, 22.9, 22.8, 21.3, 20.9, 20.1, 20.0, 16.5.

### 7-chloro-1-methyl-5-(o-tolyl)-1,3-dihydro-2H-benzo[e][1,4]diazepin-2-one (6i) from diazepam

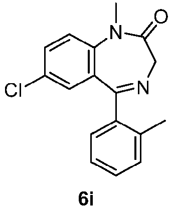

The title compound was synthesised as outlined in General Procedure E, using diazepam (57.0 mg, 0.2 mmol) and [Ru(OH₂)(*t*BuCN)₅](BF₄)₂ **2** (21.2 mg, 0.03 mmol, 10 mol%). After this time, quantitative ¹H NMR using nitromethane as internal standard indicated conversion to product **4i** of 77%. Purification using flash column chromatography eluting with 25-35% EtOAc in hexane afforded the title product as a white solid (45.0 mg, 75 %).

¹H NMR (400 MHz, CDCl₃) δ 7.47 (dd, *J =* 8.7, 2.5 Hz, 1H), 7.39 - 7.31 (m, 2H), 7.30 - 7.23 (m, 2H), 7.18 (d, *J =* 7.1 Hz, 1H), 7.05 (d, *J =* 2.6 Hz, 1H), 4.86 (d, *J =* 11.0 Hz, 1H), 3.81 (d, *J* = 11.0 Hz, 1H), 3.43 (s, 3H), 1.98 (s, 3H).

¹³C NMR (101 MHz, CDCl₃) δ 170.9, 170.0, 141.8, 138.8, 136.4, 131.9, 131.6, 131.5, 131.0, 129.9, 129.8, 129.1, 126.1, 122.7, 56.9, 34.9, 20.1.

### General Procedure F: Ru-catalysed meta-alkylation of DG-containing arenes with tertiary alkyl halides

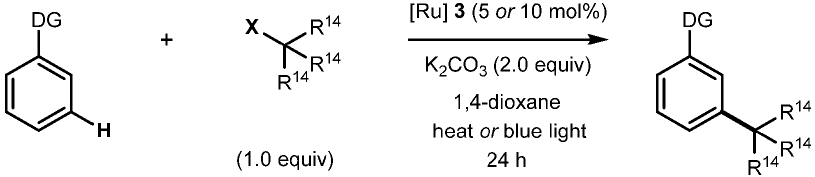

Each R¹⁴ is independently a substituent, e.g. an optionally substituted C₁₋₁₂ alkyl group.

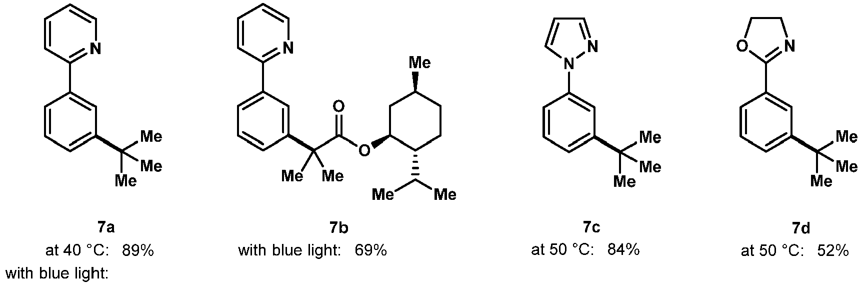

The blue light has a wavelength of 440 nm.

### General Procedure G: Ru-catalysed H / D exchange

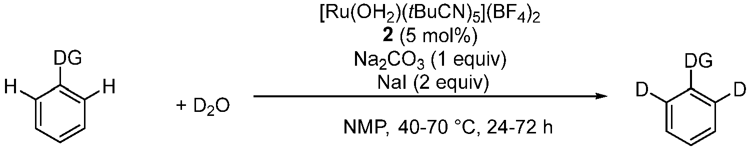

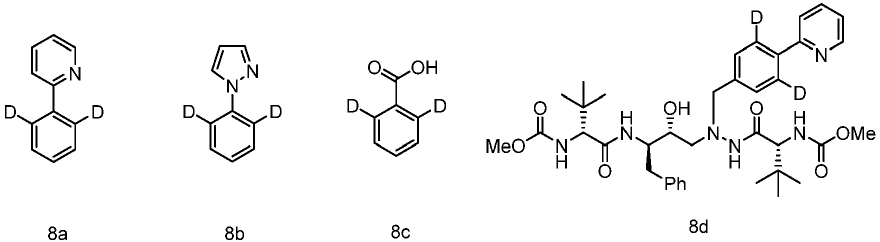

### General Procedure H: Oxidative alkene cleavage

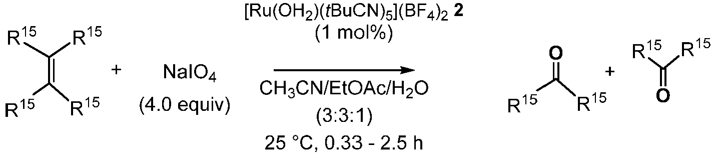

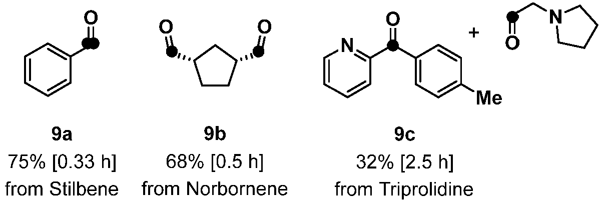

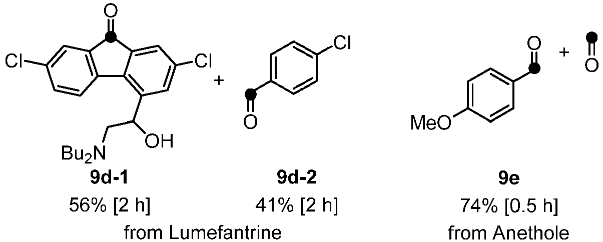

● = C atom of the cleaved C=C bond

Each R¹⁵ is independently a substituent, e.g. an optionally substituted C₁₋₁₂ alkyl or optionally substituted aryl (e.g. phenyl) or heteroaryl and two or more R¹⁵ groups may be linked to form a monocyclic or polycyclic ring.

### General Procedure I: Transfer hydrogenation

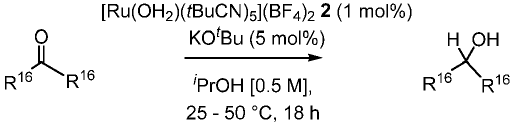

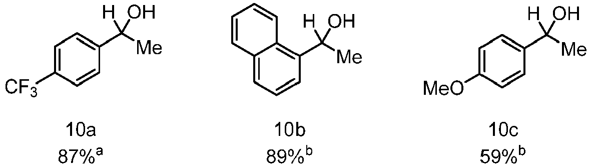

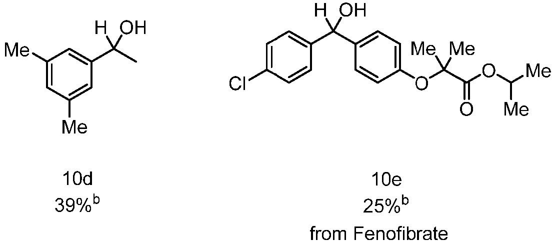

Each R¹⁶ is independently a substituent, e.g. an optionally substituted C₁₋₁₂ alkyl or optionally substituted aryl (e.g. phenyl) or heteroaryl.

### Intramolecular cyclisation

Compounds of Formula (I) may catalyse intramolecular cyclisation by reaction between a C-H of an sp²-hybridised aromatic or non-aromatic carbon atom and a primary, secondary or tertiary alkyl halide. Preferably, the cyclisation forms a 5- or 6-membered carbocyclic or heterocyclic ring.

An exemplary intramolecular cyclisation is:

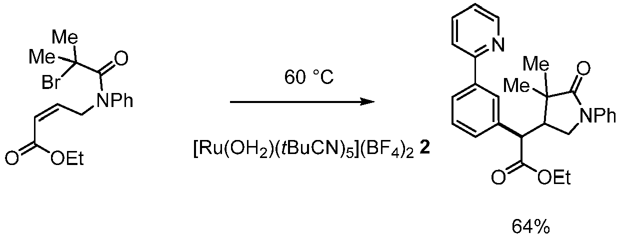

### Alkene isomerisation

Compounds of Formula (I) may catalyse alkene isomerisation, for example:

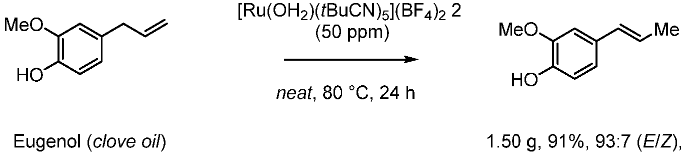

### Alkene isomerisation

Compounds of Formula (I) may catalyse hydroalkynylation of an alkyne, for example:

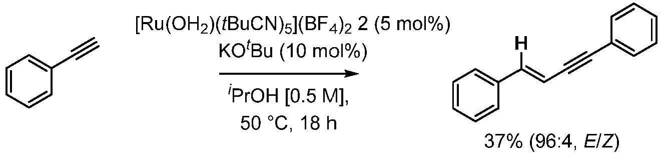

### C(sp³)-H oxidation

Compounds of Formula (I) may catalyse oxidation by an oxidising agent of C-H to C-OH for a C-H group having an sp³-hybridised carbon atom, for example:

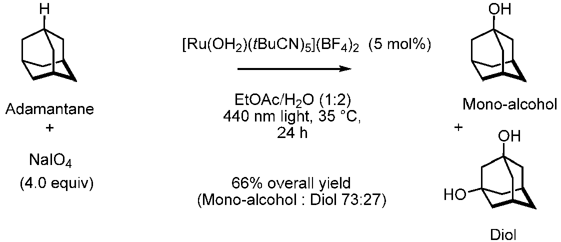

### C(sp³)-H amidation

Compounds of Formula (I) may catalyse intramolecular or intermolecular amidation of a C-H group having an sp³-hybridised carbon atom, for example:

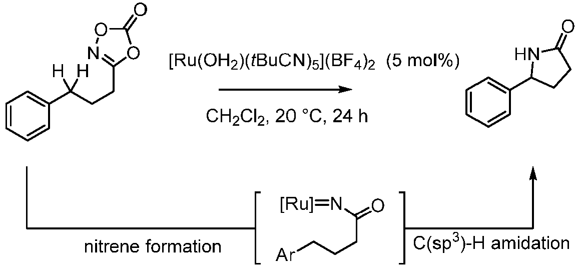

### Air stability

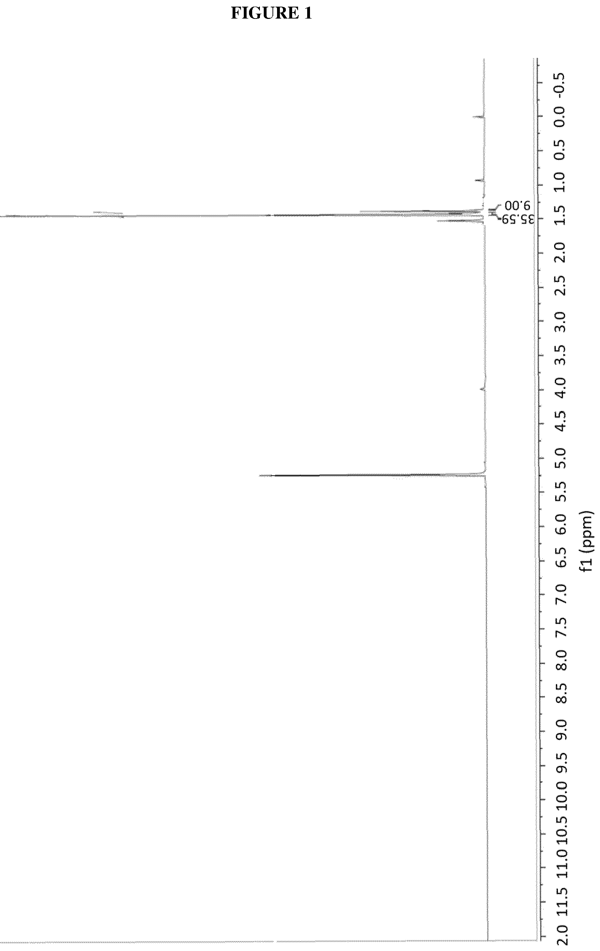

The ¹H NMR spectrum of Ru complex **2** following several weeks of storage in an ambient environment is shown in Figure 1. No significant change in the spectrum or the visible appearance of this complex was observed upon air exposure. The ability of this complex to catalyse C-H bond to C-C bond conversion as described herein was unaffected by air exposure.

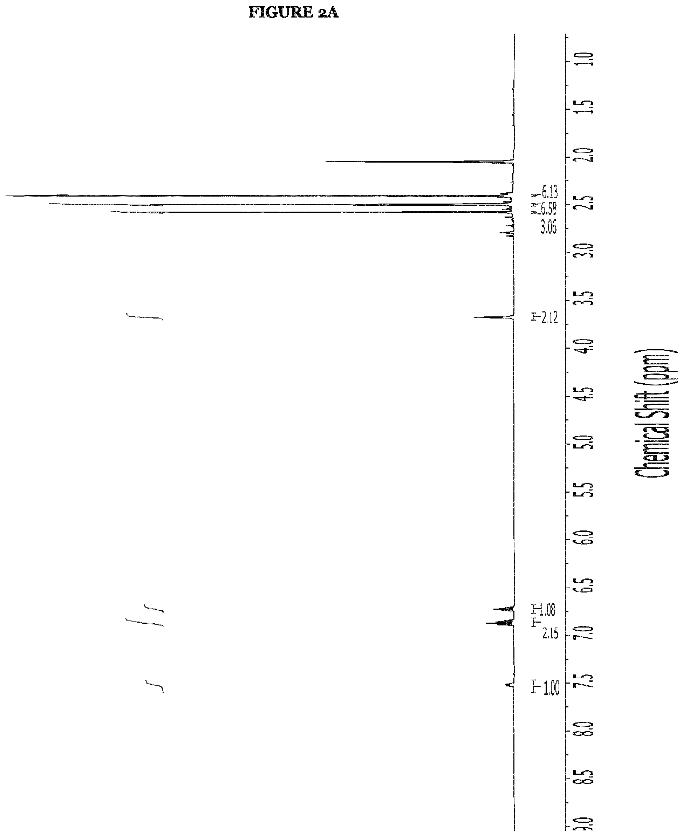

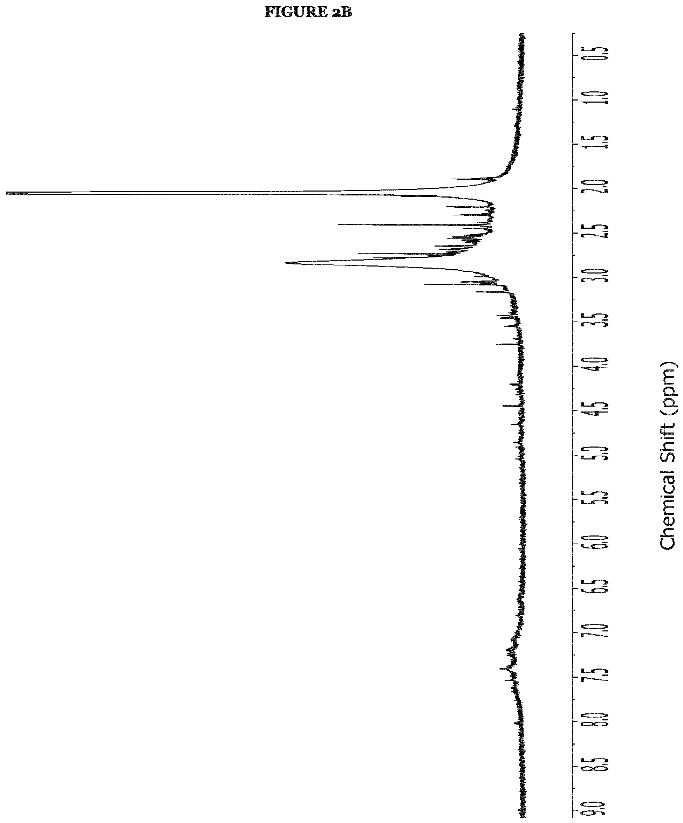

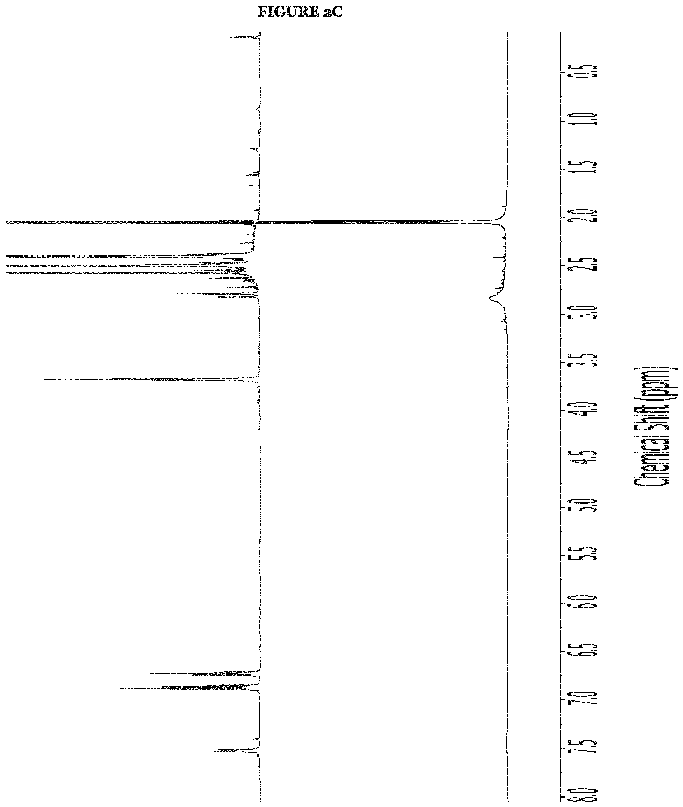

Figures 2A and 2B show the ¹H NMR spectra of Comparative Compound 1, illustrated below, before and after exposure to air. Significant differences in the spectra are apparent following air exposure, as can be seen from the stacked spectra of Figure 2C. The appearance of Comparative Compound 1 changed from an off white solid to a black solid upon exposure to the ambient environment. Attempts to convert a C-H bond to a C-C bond catalysed by Comparative Compound 1 after air exposure were unsuccessful.

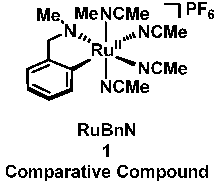

Comparative Compound 1 is disclosed as "Ru9" in Simonetti, M., Cannas, D.M., Just-Baringo, X., Citorica-Yrezabal I.J. and Larossa I. "Cyclometallated ruthenium catalyst enables late-stage directed arylation of pharmaceuticals", Nature Chem 10, 724-731 (2018).

## Claims

1. A compound of formula (I-A): 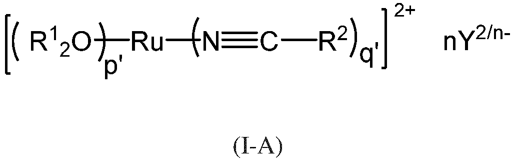
wherein R¹ in each occurrence is selected from H, optionally substituted C₁₋₁₂ alkyl and optionally substituted C₆₋₂₀ aryl;
R² in each occurrence is selected from optionally substituted C₁₋₁₂ alkyl and optionally substituted C₆₋₂₀ aryl;
p' is 1 and q' is 5 or p' is 2 and q' is 4;
p + q = 6;
Y is an anion; and
n is 1 or 2.

2. The compound according to claim 1 wherein p' is 1 and q' is 5.

3. The compound according to claim 1 or 2 wherein each R¹ is H.

4. The compound according to any one of the preceding claims wherein n is 2, optionally wherein Y is a borate, phosphate or perchlorate anion.

5. A method of forming a compound according to any one of the preceding claims, the method comprising reducing a compound of formula RuZ₃ in the presence of a compound of formula R²-CN to form an intermediate and reacting the intermediate with a compound of formula R¹₂O, wherein Z is an anionic ligand and wherein the RuZ₃ may be reduced by a reducing agent or RuZ₃ may be reduced electrochemically.

6. The method according to claim 5 wherein Z is a halide.

7. The method according to claim 5 or 6 wherein the reducing agent is zinc.

8. A method of forming a compound according to any one of claims 1-4, the method comprising reacting a compound of formula RuZp'(NC-R²)q' wherein Z is an anionic ligand with a compound of formula R¹₂O.

9. The method according to claim 8 wherein the reaction is performed the presence of a compound of formula M⁺Y⁻ wherein M⁺ is a cation.

10. A method of forming a compound of formula (C), the method comprising a reaction according to Scheme 1: 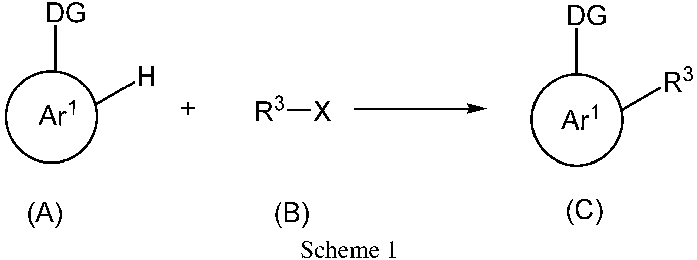 wherein (A) is a compound or a compound fragment; Ar¹ is a monocyclic or fused aromatic or heteroaromatic ring which is unsubstituted or substituted with one or more substituents; DG is a directing group; X is a leaving group; R³ is a group comprising a carbon atom bound to which X is bound, and wherein the reaction is catalysed by a compound of formula (I): 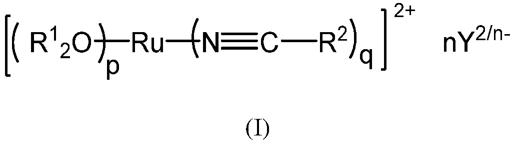
wherein R¹ in each occurrence is selected from H, optionally substituted C₁₋₁₂ alkyl and optionally substituted C₆₋₂₀ aryl;
R² in each occurrence is selected from optionally substituted C₁₋₁₂ alkyl and optionally substituted C₆₋₂₀ aryl;
p is at least 1;
q is at least 1;
p + q = 6;
Y is an anion; and
n is 1 or 2.

11. The method according to claim 10 according to Scheme 2:

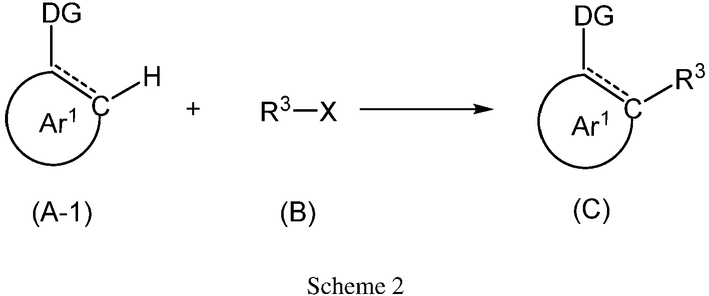

12. The method according to claim 10 or 11 wherein:
(a) the compound or compound fragment of formula (A) has formula (A-2) or (A-3): 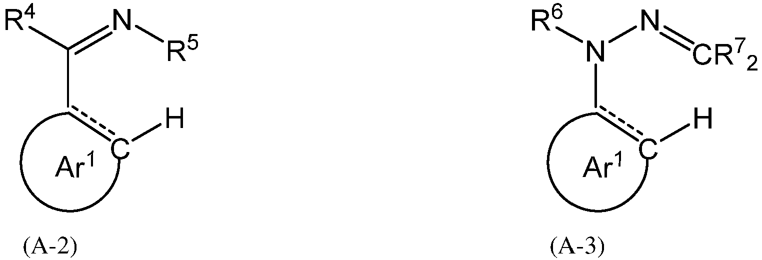 wherein R⁴-R⁷ are each independently H or a substituent, and R⁴ and R⁵ may be linked to form an optionally substituted monocyclic or fused ring, or
(b) the compound or compound fragment of formula (A) has formula (A-4): 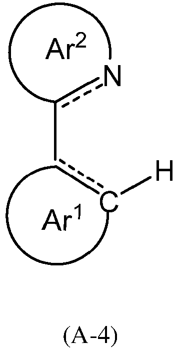 wherein Ar² is an optionally substituted moncyclic or fused heteroaromatic ring, or
(c) the compound or compound fragment of formula (A) has formula (A-5) or (A-6): 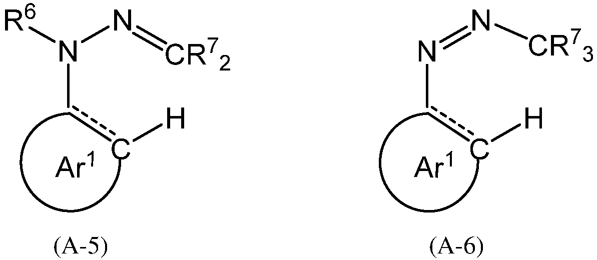 wherein R⁶ and R⁷ are each independently H or a substituent, and R⁶ and R⁷ may be linked to form an optionally substituted monocyclic or fused ring, or
(d) the compound or compound fragment of formula (A) has formula (A-7): 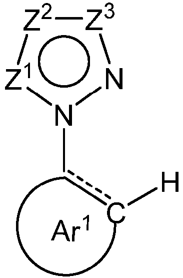 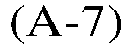 wherein Z¹, Z² and Z³ are each independently N or CR⁸ wherein R⁸ is H or a substituent.

13. The method according to claim 12 wherein Ar² of the compound of formula (A-4) has C and N ring atoms and, optionally, O or S ring atoms.

14. The method according to any one of claims 11-13 wherein X is bound to an aromatic carbon atom of an aromatic or heteroaromatic ring of R³, wherein R³ may be an optionally substituted phenyl.

15. The method according to any one of claims 11-13 wherein X is bound to an sp³ hybridised primary or secondary carbon atom of R³, wherein R³ may be an optionally substituted C₁₋₁₂ alkyl.

## Patentansprüche

1. Verbindung der Formel (I-A): 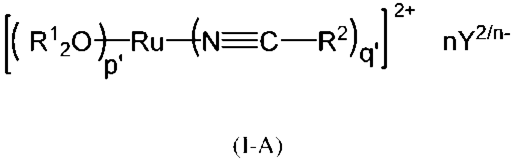
wobei R¹ bei jedem Auftreten ausgewählt ist aus H, optional substituiertem C₁₋₁₂-Alkyl und optional substituiertem C₆₋₂₀-Aryl;
R² bei jedem Auftreten ausgewählt ist aus optional substituiertem C₁₋₁₂-Alkyl und optional substituiertem C₆₋₂₀-Aryl;
p' 1 ist und q' 5 ist oder p' 2 ist und q' 4 ist;
p + q = 6;
Y ein Anion ist; und
n 1 oder 2 ist.

2. Verbindung nach Anspruch 1, wobei p' 1 ist und q' 5 ist.

3. Verbindung nach Anspruch 1 oder 2, wobei jedes R¹ H ist.

4. Verbindung nach einem der vorhergehenden Ansprüche, wobei n 2 ist, optional wobei Y ein Borat-, Phosphat- oder Perchlorat-Anion ist.

5. Verfahren zum Ausbilden einer Verbindung nach einem der vorhergehenden Ansprüche, wobei das Verfahren Reduzieren einer Verbindung der Formel RuZ₃ in Gegenwart einer Verbindung der Formel R²-CN, um ein Zwischenprodukt auszubilden, und Umsetzen des Zwischenprodukts mit einer Verbindung der Formel R¹₂O umfasst, wobei Z ein anionischer Ligand ist und wobei das RuZ₃ durch ein Reduktionsmittel reduziert werden kann oder RuZ₃ elektrochemisch reduziert werden kann.

6. Verfahren nach Anspruch 5, wobei Z ein Halogenid ist.

7. Verfahren nach Anspruch 5 oder 6, wobei das Reduktionsmittel Zink ist.

8. Verfahren zum Ausbilden einer Verbindung nach einem der Ansprüche 1-4, wobei das Verfahren Umsetzen einer Verbindung der Formel RuZp'(NC-R²)q', wobei Z ein anionischer Ligand ist, mit einer Verbindung der Formel R¹₂O umfasst.

9. Verfahren nach Anspruch 8, wobei die Reaktion in Gegenwart einer Verbindung der Formel M⁺Y⁻ durchgeführt wird, wobei M⁺ ein Kation ist.

10. Verfahren zum Ausbilden einer Verbindung der Formel (C), wobei das Verfahren eine Reaktion nach Schema 1 umfasst: 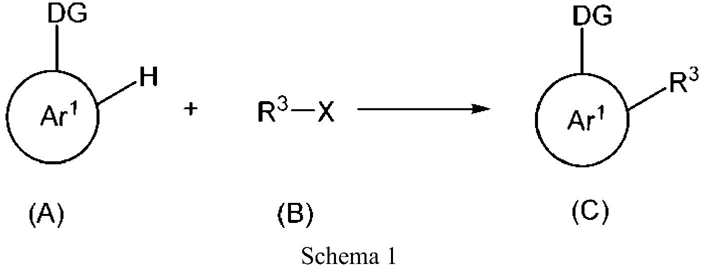 wobei (A) eine Verbindung oder ein Verbindungsfragment ist; Ar¹ ein monocyclischer oder kondensierter aromatischer oder heteroaromatischer Ring ist, der unsubstituiert oder mit einem oder mehreren Substituenten substituiert ist; DG eine dirigierende Gruppe ist; X eine Austrittsgruppe ist; R³ eine Gruppe ist, die eine Kohlenstoffatombindung umfasst, an die X gebunden ist, und wobei die Reaktion durch eine Verbindung der Formel (I) katalysiert wird: 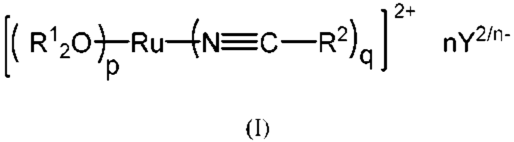
wobei R¹ bei jedem Auftreten ausgewählt ist aus H, optional substituiertem C₁₋₁₂-Alkyl und optional substituiertem C₆₋₂₀-Aryl;
R² bei jedem Auftreten ausgewählt ist aus optional substituiertem C₁₋₁₂-Alkyl und optional substituiertem C₆₋₂₀-Aryl;
p mindestens 1 ist;
q mindestens 1 ist;
p + q = 6;
Y ein Anion ist; und
n 1 oder 2 ist.

11. Verfahren nach Anspruch 10 nach Schema 2:

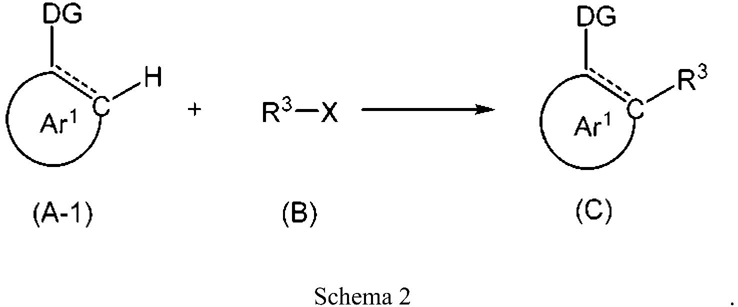

12. Verfahren nach Anspruch 10 oder 11, wobei:
(a) die Verbindung oder das Verbindungsfragment der Formel (A) die Formel (A-2) oder (A-3) aufweist: 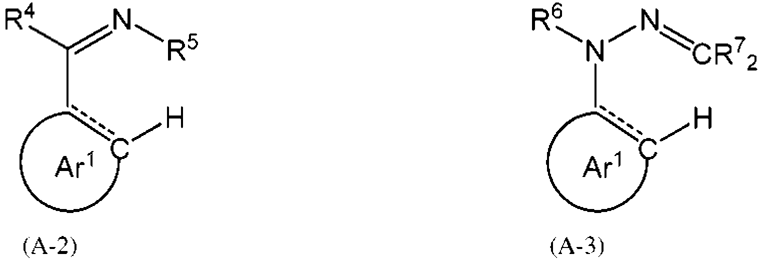 wobei R⁴-R⁷ jeweils unabhängig H oder ein Substituent sind und R⁴ und R⁵ verbunden sein können, um einen optional substituierten monocyclischen oder kondensierten Ring auszubilden, oder
(b) die Verbindung oder das Verbindungsfragment der Formel (A) die Formel (A-4) aufweist: 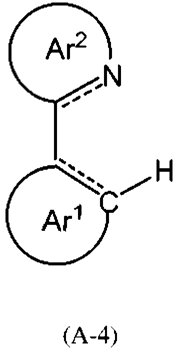 wobei Ar² ein optional substituierter monocyclischer oder kondensierter heteroaromatischer Ring ist, oder
(c) die Verbindung oder das Verbindungsfragment der Formel (A) die Formel (A-5) oder (A-6) aufweist: 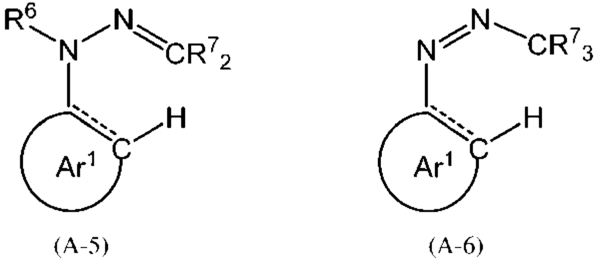 wobei R⁶ und R⁷ jeweils unabhängig H oder ein Substituent sind und R⁶ und R⁷ verbunden sein können, um einen optional substituierten monocyclischen oder kondensierten Ring auszubilden, oder
(d) die Verbindung oder das Verbindungsfragment der Formel (A) die Formel (A-7) aufweist: 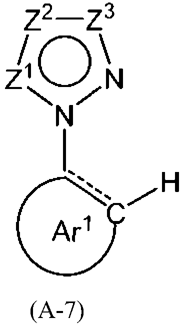 wobei Z¹, Z² und Z³ jeweils unabhängig N oder CR⁸ sind, wobei R⁸ H oder ein Substituent ist.

13. Verfahren nach Anspruch 12, wobei Ar² der Verbindung der Formel (A-4) C- und N-Ringatome und optional O- oder S-Ringatome aufweist.

14. Verfahren nach einem der Ansprüche 11-13, wobei X an ein aromatisches Kohlenstoffatom eines aromatischen oder heteroaromatischen Rings von R³ gebunden ist, wobei R³ ein optional substituiertes Phenyl sein kann.

15. Verfahren nach einem der Ansprüche 11-13, wobei X an ein sp³⁻hybridisiertes primäres oder sekundäres Kohlenstoffatom von R³ gebunden ist, wobei R³ ein optional substituiertes C₁₋₁₂-Alkyl sein kann.

## Revendications

1. Composé de formule (I-A) : 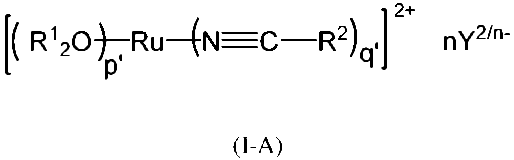
dans lequel R¹ dans chaque occurrence est choisi parmi H, un alkyle en C₁₋₁₂ éventuellement substitué et un aryle en C₆₋₂₀ éventuellement substitué ;
R² dans chaque occurrence est choisi parmi un alkyle en C₁₋₁₂ éventuellement substitué et un aryle en C₆₋₂₀ éventuellement substitué ;
p' vaut 1 et q' vaut 5 ou p' vaut 2 et q' vaut 4 ;
p + q = 6 ;
Y est un anion ; et
n vaut 1 ou 2.

2. Composé selon la revendication 1, dans lequel p' vaut 1 et q' vaut 5.

3. Composé selon la revendication 1 ou 2, dans lequel chaque R¹ est H.

4. Composé selon l'une quelconque des revendications précédentes, dans lequel n vaut 2, éventuellement dans lequel Y est un anion borate, phosphate ou perchlorate.

5. Procédé de formation d'un composé selon l'une quelconque des revendications précédentes, le procédé comprenant la réduction d'un composé de formule RuZ₃ en présence d'un composé de formule R²-CN afin de former un intermédiaire et la réaction de l'intermédiaire avec un composé de formule R¹₂O, dans lequel Z est un ligand anionique et dans lequel RuZ₃ peut être réduit par un agent réducteur ou RuZ₃ peut être réduit électrochimiquement.

6. Procédé selon la revendication 5, dans lequel Z est un halogénure.

7. Procédé selon la revendication 5 ou 6, dans lequel l'agent réducteur est le zinc.

8. Procédé de formation d'un composé selon l'une quelconque des revendications 1 à 4, le procédé comprenant la réaction d'un composé de formule RuZp' (NC-R²)q', dans lequel Z est un ligand anionique, avec un composé de formule R¹₂O.

9. Procédé selon la revendication 8, dans lequel la réaction est effectuée en présence d'un composé de formule M⁺Y⁻, dans lequel M⁺ est un cation.

10. Procédé de formation d'un composé de formule (C), le procédé comprenant une réaction selon le Schéma 1 : 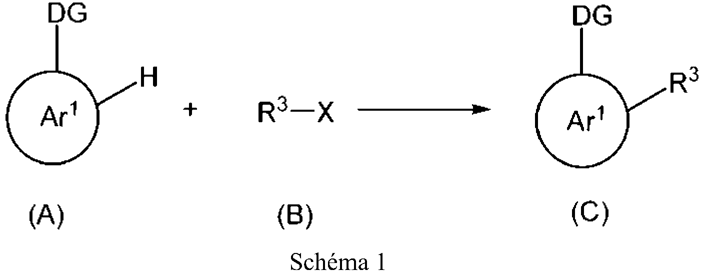 dans lequel (A) est un composé ou un fragment de composé ; Ar¹ est un cycle aromatique ou hétéroaromatique monocyclique ou fusionné qui est non substitué ou substitué par un ou plusieurs substituants ; DG est un groupe directeur ; X est un groupe partant ; R³ est un groupe comprenant un atome de carbone lié auquel X est lié, et dans lequel la réaction est catalysée par un composé de formule (I) : 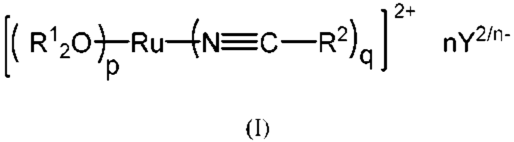
dans lequel R¹ dans chaque occurrence est choisi parmi H, un alkyle en C₁₋₁₂ éventuellement substitué et un aryle en C₆₋₂₀ éventuellement substitué ;
R² dans chaque occurrence est choisi parmi un alkyle en C₁₋₁₂ éventuellement substitué et un aryle en C₆₋₂₀ éventuellement substitué ;
p vaut au moins 1 ;
q vaut au moins 1 ;
p + q = 6 ;
Y est un anion ; et
n vaut 1 ou 2.

11. Procédé selon la revendication 10 selon le Schéma 2 :

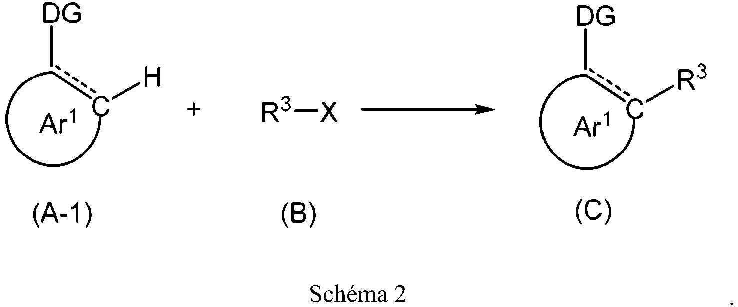

12. Procédé selon la revendication 10 ou 11, dans lequel :
(a) le composé ou le fragment de composé de formule (A) présente la formule (A-2) ou (A-3) : 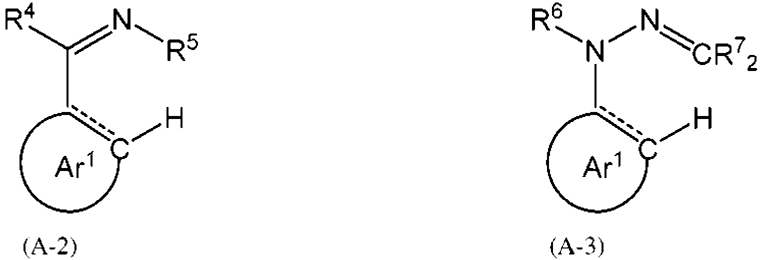 dans lequel R⁴-R⁷ sont chacun indépendamment H ou un substituant, et R⁴ et R⁵ peuvent être liés pour former un cycle monocyclique ou fusionné éventuellement substitué, ou
(b) le composé ou le fragment de composé de formule (A) présente la formule (A-4) : 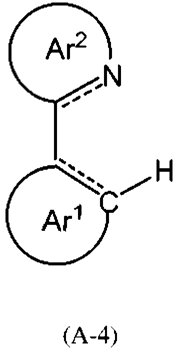 dans lequel Ar² est un cycle hétéroaromatique monocyclique ou fusionné éventuellement substitué, ou
(c) le composé ou le fragment de composé de formule (A) présente la formule (A-5) ou (A-6) : 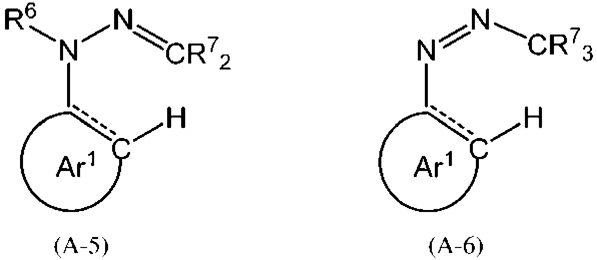 dans lequel R⁶ et R⁷ sont chacun indépendamment H ou un substituant, et R⁶ et R⁷ peuvent être liés pour former un cycle monocyclique ou fusionné éventuellement substitué, ou
(d) le composé ou le fragment de composé de formule (A) présente la formule (A-7) : 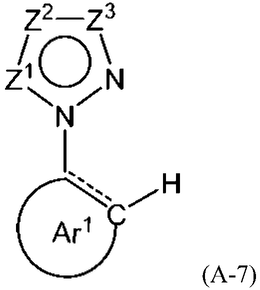 dans lequel Z¹, Z² et Z³ sont chacun indépendamment N ou CR⁸, dans lequel R⁸ est H ou un substituant.

13. Procédé selon la revendication 12, dans lequel Ar² du composé de formule (A-4) comporte des atomes de cycle C et N et, éventuellement, des atomes de cycle O ou S.

14. Procédé selon l'une quelconque des revendications 11 à 13, dans lequel X est lié à un atome de carbone aromatique d'un cycle aromatique ou hétéroaromatique de R³, dans lequel R³ peut être un phényle éventuellement substitué.

15. Procédé selon l'une quelconque des revendications 11 à 13, dans lequel X est lié à un atome de carbone primaire ou secondaire hybridé sp³ de R³, dans lequel R³ peut être un alkyle en C₁₋₁₂ éventuellement substitué.
